# EUROPEAN PATENT APPLICATION

(11) **EP 1 493 732 A1**
(43) Date of publication of application: **05.01.2005**
(21) Application number: 03710314.0
(22) Date of filing: 12.03.2003
(51) Int. Cl.: C07D 211/46, C07D 487/04, A61K 31/445, A61K 31/55, A61P 11/06, A61P 13/12, A61P 17/00, A61P 17/06, A61P 21/04, A61P 25/00, A61P 29/00, A61P 37/02, A61P 37/08, A61P 43/00

(54) **COMPOUNDS CAPABLE OF INHIBITING IMMUNOCYTE-RELATED ALLERGIC IMMUNE REACTIONS**

(30) Priority: 12.03.2002 JP 2002067271
(71) Applicant: Y'S Therapeutics Co., Limited, Tokyo 150-0021 (JP)
(72) Inventor: MORIMOTO, Chikao, Setagaya-ku, Tokyo 156-0043 (JP)
(74) Representative: Grünecker, Kinkeldey, Stockmair & Schwanhäusser Anwaltssozietät
(86) International application number: PCT/JP2003/002917
(87) International publication number: WO 2003/076399

(57) **Abstract**

The present invention is based on novel discoveries relating to ebastine, carebastine, and epinastine hydrochloride. Specifically, the present invention relates to the use of ebastine and carebastine as an inhibitor of (A) T cell proliferation, (B) Th2 type cytokine production, (C) inflammatory cytokine production, and (D) T cell migration. In addition, it relates to the use of epinastine hydrochloride as an inhibitor of (A) T cell proliferation, (B) Th2 type cytokine production, and (C) Th1 type cytokine production.

## Description

### Technical Field

The present invention relates to a novel application of ebastine, carebastine, and epinastine hydrochloride.

### Background Art

Allergic diseases are caused by overreaction of the immune system. Basic allergic reactions are generally classified into four types depending on their mechanism. Type I (immediate) allergy, also called anaphylaxis, mainly involve IgE antibodies. Damage to one's own tissue (self-tissue damages) (type II allergy) due to abnormal antibodies (autoantibodies) that can react with one's own cells or tissues involve IgG, IgM, IgA, etc. Immune complex disease is caused by type III allergy that involve antigen-antibody complexes comprising antibodies such as IgG, IgM, and IgA, antigens, and complements. Type IV (delayed type) allergy does not involve antibodies but is caused by reactions between T cells and T cell-derived inflammatory substances. In addition, among type II allergy, those that involve receptor-stimulated immunoglobulins are also called type V allergy.

Type I (immediate) allergy represented by urticaria or allergic rhinitis require the production of antigen-specific IgE in advance. Foreign antigens entering the body are first incorporated into antigen presenting cells such as macrophages and dendritic cells.. These antigens are degraded into peptides of 11 to 13 amino acids in endosomes, presented on the cell membrane by binding to MHC class II molecules, and are then recognized by T cells. When a T cell recognizes a complex of MHC and a peptide via the T cell antigen receptor (TCR) , a signal is transmitted into the cell activating them to produce various cytokines. B cells differentiate and maturate into plasma cells due to the antigen stimulation and produce antibodies (immunoglobulins) that are specific for particular antigen (s). In the peripheral tissue, depending on the environment, naive T cells differentiate into Th1 type or Th2 type subsets. Cytokines IL-4 and IL-5 that are produced by the Th2 type subset act on B cells to direct the production of IgE and IgG1.

The binding of a foreign antigen-specific IgE antibody produced by a B cell to a high-affinity IgE receptor (FcεRI) of a mast cell or basophil alone induces transmission of activation signal into the nucleus, enhances the expression of FcεRI, and suppresses apoptosis to extend the lifetime of the mast cell or basophil. Moreover, when a foreign antigen binds to FcεRI, degranulation arises and histamine and leukotriene are released that act on the surrounding tissues (exalting vascular permeability, contracting smooth muscle, etc.), thereby causing various symptoms of allergy. However, recent studies have indicated chronic inflammation as the main condition of the diseases in many type I allergic diseases such as bronchial asthma.

Chronic allergic inflammations that follow immediate reaction are caused by the migration and infiltration into the inflammation area of inflammation-related cell group, such as eosinophils, neutrophils, and lymphocytes that are activated by cytokines that are mainly released by local mast cells. The role of lymphocytes is thought to be indispensable in prolonging the inflammation of immediate allergic reactions.

T-cell activation by antigen induces phosphorylation of intracellular protein and elevation of intracellular calcium concentration at an early stage and is complete within several minutes from the antigen stimulation. After 24 to 48 hours, various activation antigen molecules and IL-2 receptor are expressed on the T cell surface, and cytokines are copiously produced and released to trigger cell proliferation reactions.

Hypersensitive allergic immune response is induced and continued by many types of inflammatory cells through the production of soluble factors, such as cytokines and intercellular interactions. In particular, helper T cells (hereinafter abbreviated as "Th cells") are thought to be the effector cells and play an important role in controlling hypersensitive reactions. Two subsets of helper T cells (CD4⁺ T cells) have been identified based on their cytokine production profile (Kapsenberg M, Wierenga E, Bos J, Jansen H. "Functional subsets of allergen-reactive human CD4⁺ T cells." Immunol Today. 12:392-395, 1991; Romagnani S. "Lymphokine production by human T cell in disease states." Ann Rev Immunol. 12:227-257, 1994).

One is the T helper cell type 1 (Th1) which releases IL-2, IFN-γ, and so on (Kapsenberg M. *et al*., 1991, supra; Romagnani S., 1994, *supra*). The other is the T helper cell type 2 (Th2) which releases IL-4, IL-5, and so on (Kapsenberg M. *et al*., 1991, *supra*; Romagnani S., 1994, *supra*). Inflammatory cytokines (IL-6, TNF-α, etc.) are also produced by macrophages and the like in addition to T cells.

IL-4 acts on B cells to promote class switching, thereby playing an important role in IgE production. On the other hand, IL-2 and IFN-γ suppress IgE production enhancing the action of IL-4 in a different manner respectively. In addition to cytokines, stimulation of the B cell surface molecule CD40 is important in inducing the production of IgE. During this process, B cell proliferation is induced by IL-6, and IL-5 acts in an auxiliary manner. Furthermore, IL-5 specifically induces the differentiation and proliferation of eosinophils, and promotes mediator secretion from eosinophils and basophils. In several allergic diseases, it has been reported that Th2 cells preferentially accumulate at inflammatory sites and induce hypersensitive reactions (Del Prete GF, De Carli M, D'Elios MM *et al*. "Allergen exposure induces the activation of allergen-specific Th2 cells in the airway mucosa of patients with allergic respiratory disorders." Eur J Immunol. 23:1445-1449, 1993; Robinson D, Hamid Q, Bentley A, Ying S, Kay AB, Durham SR. "Activation of CD4⁺ T cells, increased Th-2 type cytokine mRNA expression, and eosinophil recruitment in bronchoalveolar lavage after allergen inhalation challenge in patients with atopic asthma." J Allergy Clin Immunol. 92:313-324, 1993; Maggi E, Biswas P, Del Prete GP Parronchi P, Nacchia D, Simonelli C, Emmi L, Decarli M, Tiri A, Ricci M, *et al*. "Accumulation of Th2-like helper T cells in the conjunctiva of patients with vernal conjunctivitis." J Immunol. 146: 1169-1174, 1991). Therefore, it is considered possible to treat such allergic diseases and hypersensitivity by specifically inhibiting the function of Th2 cells.

Macrophages not only present antigens in the initial phase, but also get self-activated to actively contribute to the proliferation, differentiation, and activation of neighboring cells via production of inflammatory cytokines (IL-6, TNF-α, and so on). These various cytokines produced by macrophages also play important roles in allergic reactions.

The migration and infiltration of inflammatory cells at inflammatory sites have important roles in allergic reactions. The lymphocyte extravasation consists of three steps, i.e., the cell adhesion cascade: (1) rolling, (2) strong adhesion, and (3) transmigration. Each of the steps is mediated by the binding of adhesive molecules and ligands expressed on the cell membranes of leukocytes and vascular endothelial cells, respectively.

The adhesive molecules are structurally categorized into a large number of families: the integrin family, the immunoglobulin superfamily, the selectin family, the cadherin family, the link protein family, the sialomucin family, etc.

Surface molecules present on activated T cells, such as CD11a, CD29, CD44, CD26, and CD47, have a close relationship with cell adhesion. CD11a has been revealed to be the α chain of LFA-1. LFA-1 is expressed only in lymphocytes within lymphatic tissue, and is associated with intercellular adhesion via the binding with its ligand ICAM-1. Among the β1 integrin family members, VLA-4 (CD4.9d/CD29) is particularly involved in the adhesion of T cells, and one of its ligands is VCAM-1 that is expressed on vascular endothelial cells. CD44 is a hyaluronic acid receptor that is associated with the adhesion of lymphocytes to endothelial cells and interstitial cells at inflammatory sites. CD26 is expressed on memory T cells and is associated with the migration of T cells (Hafler DA, Fox DA, Manning ME, Schlossman SF, Reinherz EL, Weiner HL. "*In vivo* activated T lymphocytes in the peripheral blood and cerebrospinal fluid of patients with multiple sclerosis." N Engl J Med. 312:1405, 1985; Nakao H, Eguchi K, Kawakami A, Migita K, Otsubo T, Ueki Y, *et al*. "Increment of Ta1 positive cells in peripheral blood from patients with rheumatoid arthritis." J Rheumatol. 16:904, 1989). CD47 is called integrin-associated protein and is mainly involved in cell migration (Masuyama J, Berman JS, Cruikshank WW, Morimoto C, Center DM. "Evidence for recent as well as long term activation of T cells migrating through endothelial cell monolayers in vitro." J Immunol. 148:1367, 1992; Ohashi Y, Iwata S, Kamiguchi K, Morimoto C. "Tyrosine phosphorylation of Crk-associated substrate lymphocyte-type is a critical element in TCR- and beta 1 integrin-induced T lymphocyte migration." J Immunol. 163:3727, 1999; Liu Y, Merlin D, Burst SL, Pochet M, Madara JL, Parkos CA. "The role of CD47 in neutrophil transmigration. Increased rate of migration correlates with increased cell surface expression of CD47." J Biol Chem. 276:40156, 2001).

Moreover, β1 integrin is not only involved in adhesion and migration of cells but also has various biological functions including activation and proliferation, and cytokine production of T cells. Both β1 integrins and extracellular matrix accumulate upon binding of the two, followed by accumulation of intracellular tyrosine kinase (FAK, Src), adapter proteins (p130Cas, paxillin), and actin-binding proteins (α-actinin, vinculin, talin) (Schaller MD, Otey CA, Hildebrand JD, Parsons JT. "Focal adhesion kinase and paxillin bind to peptides mimicking beta integrin cytoplasmic domains." J Cell Biol. 130:1181, 1995; Lewis JM, Schwartz MA. "Mapping in vivo associations of cytoplasmic proteins with integrin beta 1 cytoplasmic domain mutants." Mol Biol Cell. 6(2):151, 1995). These accumulated protein aggregates bind to actin fibers to form focal adhesions that repeatedly activate target molecules through enzyme reactions such as tyrosine phosphorylation, in order to control biological functions via generation of various signals.

The present inventors have reported strong tyrosine phosphorylation of a 105-kDa protein (pp105) in T cells through the stimulation mediated by β1 integrin (Nojima Y, Rothstein DM, Sugita K, Schlossman SF, Morimoto C. "Ligation of VLA-4 on T cells stimulates tyrosine phosphorylation of a 105-kD protein." J Exp Med. 175:1045, 1992). Through cDNA cloning, pp105 was revealed to be a homologue of Crk-associated substrate. Thus, this protein was named Crk-associated substrate lymphocyte type (Cas-L) (Minegishi M, Tachibana K, Sato T, Iwata S, Nojima Y, Morimoto C. "Structure and function of Cas-L, a 105-kD Crk-associated substrate-related protein that is involved in beta 1 integrin-mediated signaling in lymphocytes." J Exp Med. 1:1365, 1996; Law SF, Estojak J, Wang B, Mysliwiec T, Kruh G, Golemis EA. "Human enhancer of filamentation 1, a novel p130-like docking protein, associates with focal adhesion kinase and induces pseudohyphal growth in *Saccharomyces cerevisiae."* Mol Cell Biol. 16:3327, 1996). In addition, Cas-L was found to be directly tyrosine phosphorylated by FAK and Src family tyrosine kinases (Tachibana K, Urano T, Fujita H, Ohashi Y, Kamiguchi K, Iwata S, *et al*. "Tyrosine phosphorylation of Crk-associated substrates by focal adhesion kinase. A putative mechanism for the integrin-mediated tyrosine phosphorylation of Crk-associated substrates." J Biol Chem. 272:29083, 1997). Cas-L is expressed in lymphatic cells such as peripheral blood T cells and B cells, and thymus cells. Cas-L is tyrosine phosphorylated by v-Src, BCR-ABL, and v-Crk molecules, and binds to adapter proteins such as Crk and Nck, tyrosine kinases such as Src, and phosphatases such as SH-PTP2 via their SH2 domains. Tyrosine phosphorylation of Cas-L is essential for T cell activation mediated by β1 integrin and plays an important role in T cell proliferation, cytokine production, cell migration, and cell adhesion (Ohashi Y, Iwata S, Kamiguchi K, Morimoto C. "Tyrosine phosphorylation of Crk-associated substrate lymphocyte-type is a critical element in TCR- and beta 1 integrin-induced T lymphocyte migration." J Immunol. 163: 3727, 1999; Kamiguchi K, Tachibana K, Iwata S, Ohashi Y, Morimoto C. "Cas-L is required for beta 1 integrin-mediated costimulation in human T cells. " J Immunol. 163:563, 1999). Accordingly, studies of tyrosine phosphorylation of β1 integrin-related molecules represented by Cas-L are thought to be important in the evaluation of T cell functions such as cell adhesion and cell migration.

Histamine is an in vivo amine intracellularly synthesized from L-histidine by a decarboxylase,, a compound isolated by Dale in 1910 from ergot. Histamine is mainly stored within granules of mast cells and basophils., It is released by various physical and chemical stimuli and the cross-link formation of IgE-FcεRI complex by antigens. Various physiological activities of histamine including immune-related reactions in target tissue such as acceleration of vascular permeability, contraction of smooth muscles, vasodilation, and elevation of mucus secretion, as well as acceleration of gastric acid secretion from gastric parietal cells have been well studied. Recently, histamine has been found to act as an important neurotransmitter in the central nervous system. These physiological activities are expressed via a histamine receptor.

Four subtypes of histamine receptors (H1, H2, H3, and H4) have currently been identified. The H1 receptor is mainly expressed on tissues such as capillaries, vascular smooth muscles, vascular endothelia, bronchial smooth muscles, intestinal tract smooth muscles and so on. It causes typical immediate allergic symptoms via the binding of histamines released from mast cells. The H2 receptor that is mainly expressed on gastric parietal cells and airway goblet cells, secrete gastric acid and airway mucus due to histamine stimulation. Within the central nervous system, there is a nervous system called the histamine nervous system where the histamine receptor subtypes H1, H2, and H3 are expressed. In addition to the nerve cells, the H1 and H2 receptors are also expressed on glial cells. The H1 receptor of the histamine nervous system mediate various central functions including awakening, appetite, drinking, body temperature regulation, regulation of the sense of equilibrium, regulation of neuroendocrine, and suppression of convulsions. The main side effect of histamine H1 receptor antagonists (i.e., H1 blockers) is drowsiness, because the H1 receptor in the brain controls awakening. The H3 receptor is mainly expressed in the central nervous system and has been identified as an autoreceptor that controls histamine release from histamine nerve endings. Moreover, it is also expressed on the nerve endings of nerve systems other than the histamine nerve system, and is suggested to be involved in the suppression of transmitter release. The expression of the H4 receptor has been confirmed in peripheral tissues and their functions are currently being analyzed.

Histamine has recently been reported to participate in the regulation of allergy and inflammation (Jutel M, Watanabe T, Klunker S, Akdis M, Thomet OA, Malolepszy J, *et al*. "Histamine regulates T-cell and antibody responses by differential expression of H1 and H2 receptors." Nature. 413:420-5, 2001; Jutel M, Klunker S, Akdis M, Malolepszy J, Thomet OA, Zak-Nejmark T, *et al*. "Histamine upregulates Th1 and downregulates Th2 responses due to different patterns of surface histamine 1 and 2 receptor expression." Int Arch Allergy Immunol. 124(1-3):190-2, 2001; Lagier B, Lebel B, Bousquet J, Pene J. "Different modulation by histamine of IL-4 and interferon-gamma (IFN-gamma) release according to the phenotype of human Th0, Th1 and Th2 clones. " Clin Exp Immunol. 108(3):545-51, 1997; Horvath BV, Szalai C, Mandi Y, Laszlo V, Radvany Z, Darvas Z, *et al*. "Histamine and histamine-receptor antagonists modify gene expression and biosynthesis of interferon-gamma in peripheral human blood mononuclear cells and in CD19-depleted cell subsets." Immunol Lett. 70 (2) :95-9, 1999). The H2 receptor is present mainly on human T cells (Sachs B, Hertl M, Merk HF. "Histamine receptors on lymphocytes, distribution and functional significance." Skin Pharmacol Appl Skin Physiol. 13 (6) : 313-23, 2000). However, histamine H1 and H2 receptors are present on mouse T cells and are reported to be involved in signal transduction for promoting immune activation and suppressing immune activation, respectively. A hypothesis has been proposed that histamine enhances Th1 cytokine production from T cells via the H1 receptor and at the same time suppresses both Th1 and Th2 cytokine production from T cells via the H2 receptor (Jutel M, *et al*., Nature, 2001, supra).

Histamine H1 receptor antagonists developed as agents to treat immediate allergy strongly bind to the H1 receptor of target cells, preventing binding of histamine to the receptor and suppressing allergic symptoms. In addition, they also have various pharmacological effects such as local anesthetic effects, central nervous system sedative effects, anti-arrhythmic effects, atropine-like effects, anti-serotonin effects, and anti-kinin effects. Aiming to reduce side effects and increase drug compliance, hitherto, a large number of first- and second-generation H1 blockers (antihistamines) have been developed. The second-generation H1 blockers have less sedative effects such as drowsiness and anti-cholinergic effects. Therefore, they are presently widely used as therapeutic agents for type I allergic diseases such as urticaria and allergic rhinitis (Storms WW. "Clinical studies of the efficacy and tolerability of ebastine 10 or 20 mg once daily in the treatment of seasonal allergic rhinitis in the US." Drugs. 52 Suppl 1:20, 1996; Kalis B. "Double-blind multicentre comparative study of ebastine, terfenadine and placebo in the treatment of chronic idiopathic urticaria in adults." Drugs. 52 Suppl 1:30, 1996).

H1 blockers have an ammonium group common to their chemical structure and are recognized via this ammonium group by H1 receptors., On the other hand, other pharmacological effects are determined based on the structure other than this ammonium group. H1 blockers also bind to the H2 receptor, albeit to a lesser extent than their binding to the H1 receptor (Leurs R, Church MK, Taglialatela M. "H1-antihistamines: inverse agonism, anti-inflammatory actions and cardiac effects." Clin Exp Allergy. 32(4):489-98, 2002). In addition, a large number of histamine antagonists have been reported to show inverse agonism (Pillot C, Ortiz J, Heron A, Ridray S, Schwartz JC, Arrang JM. "Ciproxifan, a histamine H3-receptor antagonist/inverse agonist, potentiates neurochemical and behavioral effects of haloperidol in the rat." Journal of Neuroscience. 22(16):7272-80, 2002; Nonaka H, Otaki S, Ohshima E, Kono M, Kase H, Ohta K, *et al*. "Unique binding pocket for KW-4679 in the histamine H1 receptor." Eur J Pharmacol. 345:111-7, 1998). Depending on the structure of the H1 blockers, differences have been recognized in their affinity to the H1 blocker receptors and in their clinical effectiveness.

Terfenadine is an agent that can suppress the aforementioned Th2 type cytokine production. This agent is an H1 blocker that does not induce anticholinergic effects or drowsiness. However, it has been reported to infrequently cause side effects including QT interval prolongation and arrhythmia. These side effects are considered to be the result of its antagonism to histamine receptors (Roberts DJ. "A preclinical overview of ebastine. Studies on the pharmacological properties of a novel histamine H1 receptor antagonist." Drugs 52 (Suppl):8-14, 1996). However, recent researches suggest that terfenadine may have different properties that are unrelated to histamine blocking at the receptor level (Massey WA, Lichtenstein LM. "The effect of antihistamines beyond H1 antagonism in allergic inflammation." J Allergy Clin Immunol. 86:1019-1024, 1990; Crampette L, Mainprice B, Bloom M, Bousquet J, Campbell AM. "Inhibition of mediator and cytokine release from dispersed nasal polyp cells by terfenadine." Allergy. 51:346-349, 1996).

H1 blockers that similarly induce no anticholinergic effects or drowsiness include ebastine (4-diphenylmethoxy-1[3-(4-terbutyl-benzoyl)-propyl]piperidine; molecular weight 471.68). Ebastine is structurally similar to terfenadine but has almost no side effect such as QT interval prolongation and arrhythmia. Therefore, it is widely used today as a therapeutic agent for seasonal allergic rhinitis and the like (Storms WW. "Clinical studies of the efficacy and tolerability of ebastine 10 or 20 mg once daily in the treatment of seasonal allergic rhinitis in the US." Drugs 52 (Suppl. 1):20-25, 1966; Kalis B. "Double-blind multicentre comparative study of ebastine, terfenadine and placebo in the treatment of chronic idiopathic urticaria in adults." Drugs 52 (Suppl. 1):30-34, 1996). Although ebastine is effective in the treatment of allergic diseases, its Th2 type cytokine production-suppressing activity and such has not been analyzed. Hence, it may include other mechanisms.

In the initial stages of a hypersensitive response, specific allergens are recognized by T cell receptors (TcR), whereby T cells get activated and release various cytokines that induce allergy. However, this process alone cannot induce the activation of T cells. Various findings suggest the existence of a second factor called costimulation signal induced through an additional T cell surface molecule different from TcR (Robey E, Allison JP. "T-cell activation: Integration of signals from the antigen receptor and costimulatory molecule." Immunol Today. 16:306-309, 1995).

The costimulation signal is characterized by T cell proliferation, cytokine production and so on. In fact, this signal is essential for a complete activation of T cells via TcR stimulation. Therefore, the induction of a costimulation signal also plays an important role in hypersensitive immune reactions. The costimulation signal may be produced by several types of accessory molecules such as CD28/CTLA-4 (Green JM, Noel PJ, Sperling AI, Walunas TL, Leishow DJ, Stack R, Gray GS, Bluestone JA, Thompson CB. "Absence of B7-dependent responses in CD28-deficient mice." Immunity. 1:501-508, 1994; Gimmi CD, Freeman GJ, Sugita K, Freedman AS, Morimoto C, Nadler LN. "B7 provides a costimulatory signal which induces T cells to proliferate and secrete IL-2." Proc Natl Acad Sci USA. 88:6575-6579, 1991; Freeman GJ, Borriello F, Hodes RJ, Reiser H, Hathcock KS, Laszlo G, McKnight AJ, Kim J, Du L, Lombard DB, *et al*. "Uncovering of functional alternative CTLA-4 counter-receptor in B7-deficient mice." Science. 262:907-909, 1993; Linsley PS, Ledbetter JA. "The role of the CD28 receptor during T cell response to antigen." Annu Rev Immunol. 11:191-212, 1993).

Moreover, the present inventors have recently identified a novel costimulation molecule, CD26, that is expressed on CD4⁺ memory T cells (Morimoto C, Torimoto Y, Levinson G, Rudd CE, Schrieber M, Dang NH, Letvin NL, Schlossman SF. "1F7, a novel cell surface molecule involved in helper function of CD4 cells." J Immunol. 143:3430-3439, 1989; Dang NH, Torimoto Y, Deusch K, Schlossman SF, Morimoto C. "Comitogenic effect of solid-phase immobilized anti-1F7 on human CD4 T cell activation via CD3 and CD2 pathways." J Immunol. 144:4092-4100, 1990; Tanaka T, Kameoka T, Yarson A, Schlossman SF, Morimoto C. "The costimulatory activity of the CD26 antigen requires dipeptidyl peptidase IV enzyme activity." Proc Natl Acad Sci USA. 90:4586-4590, 1993). In addition, this CD26 molecule is also said to be involved in the migration of effector T cells to inflammatory sites; such migration is seen in diseases caused by immunity (Hafler DA, Fox DA, Manning ME, Schlossman SF, Reinherz EL, Weiner HL. "*In vivo* activated T lymphocytes in the peripheral blood and cerebrospinal fluid of patients with multiple sclerosis." N Engl J Med. 312:1405-1411, 1985; Nakao H, Eguchi K, Kawakami A, *et al*. "Increment of Ta1 positive cells in peripheral blood from patients with rheumatoid arthritis." J Rheumatol. 16:904-910, 1989).

### Disclosure of the Invention

The first objective of the present invention is to elucidate a novel action mechanism of ebastine (4-diphenylmethoxy-1[3-(4-terbutyl-benzoyl)-propyl]piperidine), carebastine (4-[4-[4-(di-phenylmethoxy)-1-piperidinyl]-1-oxobutyl]-α, α-di-methylbenzeneacetic acid), and epinastine hydrochloride (3-amino-9,13b-dihydro-1H-dibenz(c,f)imidazol(1,5-a)azepine hydro-chloride; molecular weight 285.78) (Roeder T, Degen J, Gewecke M. "Epinastine, a highly specific antagonist of insect neuronal octopamine receptors." Eur J Pharmacol, 349(2-3):171-177, 1998). Another objective of the present invention is to provide compounds having fewer side effects and that are able to suppress allergic immune response due to costimulation signals such as cytokine production and T cell migration as described above. Yet another objective of the present invention is to provide compositions comprising these compounds.

In order to achieve the aforementioned objectives, the present inventors attempted to analyze the effects of ebastine, carebastine, and epinastine hydrochloride on T cell proliferation and cytokine production via different costimulation signal pathways in order to analyze the allergic immune response inhibition mechanism of ebastine, carebastine, and epinastine hydrochloride. Furthermore, the effects of ebastine, carebastine, and epinastine hydrochloride on T cell migration and inflammatory cytokine production due to T cells and macrophages were analyzed. These analyses indicated that under costimulation conditions, ebastine and carebastine inhibit T cell proliferation, Th2 type cytokine production, inflammatory cytokine production, as well as T cell migration, all in a concentration-dependent manner. In addition, epinastine hydrochloride was indicated to inhibit, in a concentration-dependent manner, T cell proliferation, Th2 type cytokine production, and Th1 type cytokine production. The present invention is based on these novel findings relating to ebastine, carebastine, and epinastine hydrochloride. Specifically, the present invention provides:
[1] a compound having the formula (1): 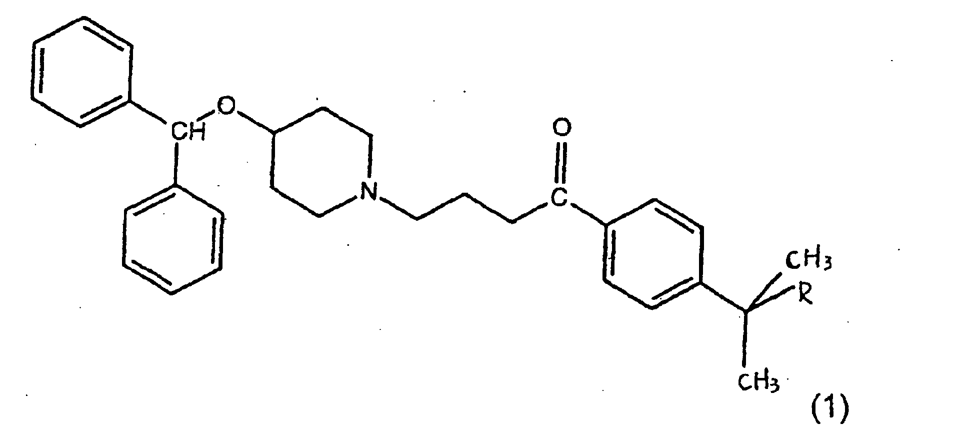 (wherein, R is CH₃ or COOH),
   that is able to suppress an allergic immune response involving an immunocyte, wherein said allergic immune response is selected from the group consisting of:
   (A) proliferation of T cell;
   (B) production of Th2 type cytokine;
   (C) production of inflammatory cytokine; and
   (D) T cell migration;
[2] a compound having the formula (2) or salts thereof: 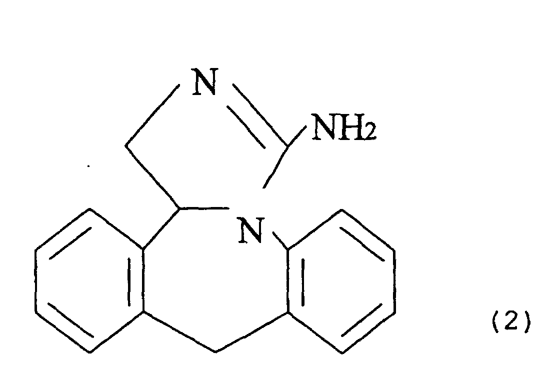 that is able to suppress an allergic immune response involving an immunocyte, wherein said allergic immune response is selected from the group consisting of:
   (A) proliferation of T cell;
   (B) production of Th2 type cytokine; and
   (C) production of Th1 cytokine;
[3] the compound according to [1], wherein the inflammatory cytokine is produced from a T cell or macrophage;
[4] a composition comprising the compound according to [1] or [3];
[5] a composition comprising the compound or salts thereof according to [2];
[6] an agent for suppressing an allergic immune response involving an immunocyte that comprises, as an active ingredient, the compound having the formula (1): 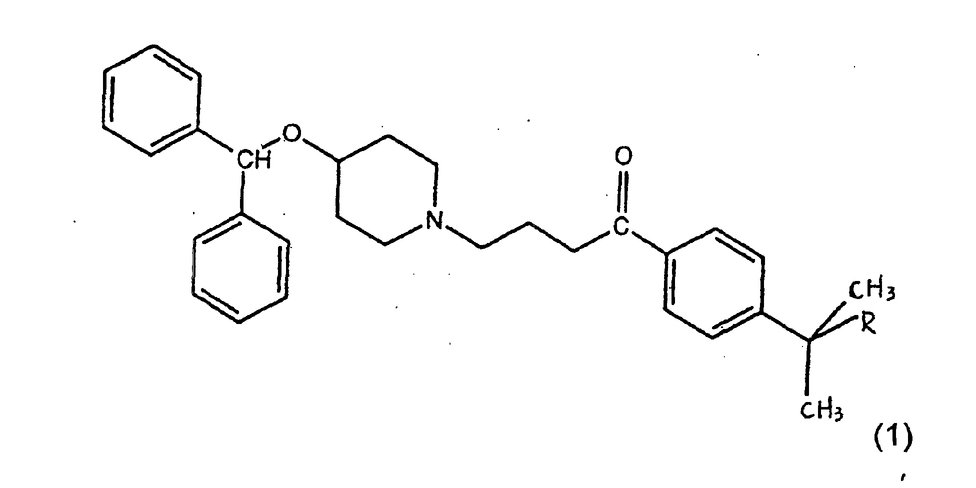 wherein said allergic immune response is selected from the group consisting of:
   (A) proliferation of T cell;
   (B) production of Th2 type cytokine;
   (C) production of inflammatory cytokine; and
   (D) T cell migration; and
[7] an agent for suppressing an allergic immune response involving an immunocyte that comprises, as an active ingredient, the compound having the formula (2) or salts thereof: 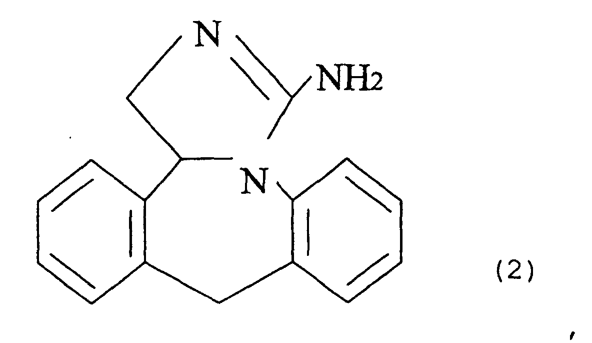
wherein said allergic immune response is selected from the group consisting of:
(A) proliferation of T cell;
(B) production of Th2 type cytokine; and
(C) production of Th1 type cytokine.

The present invention is based on the discovery of novel functions of ebastine (the compound of formula (1) above, wherein R is CH₃) and its oxidized metabolite, carebastine (the compound of formula (1) above, wherein R is COOH), and relates to the use of ebastine and carebastine as agents that suppress allergic immune response involving immunocytes other than as H1 blocker. In addition, the present invention relates to the use of ebastine and carebastine in methods for treating diseases associated with allergic immune response involving immunocytes that comprise the administration of ebastine or carebastine, or in the manufacture of agents that suppress allergic immune response involving immunocytes.

Examples of the novel functions of ebastine and carebastine include: suppression of T cell proliferation, suppression of Th2 type cytokine production, suppression of inflammatory cytokine production from T cells or macrophages, and suppression of T cell migration. That is, the present invention relates to the use of ebastine and carebastine as agents suppressing the aforementioned various allergic immune responses induced by immunocytes comprising T cells and macrophages that play a central role in allergic immune response. Herein below, each of these various functions is described in detail.

The suppression of T cell proliferation can be mentioned as the first example of the novel function of ebastine and carebastine. T cell proliferation is a phenomenon wherein the number of T cells increase through, for example, activation of T cells in the resting stage due to exogenous or autoantigen stimuli that are able to induce allergy. Such T cell proliferation can be measured in cell cultures, for example, by the intake of ³H-thymidine and so on. The *in vitro* ebastine concentration required to suppress such T cell proliferation is 1 µM to 20 µM, preferably 10 µM to 20 µM. In addition, the *in vitro* carebastine concentration required to suppress the T cell proliferation is 1 µM to 200 µM, preferably 20 µM to 200 µM.

As the second example of the novel function of ebastine and carebastine, the ability to selectively suppress the production of Th2 type cytokines can be mentioned. Herein, "selectively" means that the production of Th2 type cytokines detected at inflammatory sites in allergic diseases alone are specifically suppressed rather than suppressing the production of all cytokines from T helper cells, without affecting the production of cytokines such as IL-2 and TNF-γ from Th1 type cells. Th2 type cytokines are those produced from T helper cell type 2, which is detected at inflammatory sites of a wide range of immunoallergic diseases including bronchial asthma, atopic dermatitis, chronic graft-versus-host disease (GVHD) , systemic lupus erythematosus, and myasthenia gravis. Examples of these cytokines include IL-4, IL-5, IL-6, IL-10, and IL-13. The identification and quantification of these Th2 type cytokines can be performed by ELISA wherein antibodies against the cytokines are immobilized. The *in vitro* ebastine concentration required to suppress the production of Th2 type cytokines is 1 µM to 20 µM, preferably 5 µM to 20 µM, and even more preferably 10 µM. In addition, the *in vitro* carebastine concentration required to suppress the production of Th2 type cytokines is 1 µM to 200 µM, preferably 10 µM to 200 µM, and even more preferably 100 µM to 200 µM.

The third novel function of ebastine and carebastine is exemplified by the suppression of inflammatory cytokine production. Examples of these inflammatory cytokines typically include IL-1 and IL-6; however herein, they are not limited thereto and include TNF-α and so on. Furthermore, the inflammatory cytokines whose production is suppressed by ebastine and carebastine comprise not only those produced from macrophages but also those produced from T cells. The ebastine concentration required to suppress the production of inflammatory cytokines by T cells in an *in vitro* system is 1 µM to 20 µM, preferably 5 µM to 20 µM, and even more preferably 10 µM. Moreover, the ebastine concentration required to suppress the production of inflammatory cytokines by macrophages in an *in vitro* system is also 1 µM to 20 µM, preferably 5 µM to 20 µM, and even more preferably 10 µM. In addition, the carebastine concentration required to suppress the production of inflammatory cytokines by T cells in an *in vitro* system is 1 µM to 200 µM, preferably 10 µM to 200 µM, and even more preferably 100 µM to 200 µM. Moreover, the carebastine concentration required to suppress the production of inflammatory cytokines by macrophages in an *in vitr*o system is 1 µM to 200 µM, preferably 10 µM to 200 µM, and even more preferably 100 µM to 200 µM. The measurement of inflammatory cytokines such as IL-6 and TNF-α can be performed, for example, using an ELISA kit wherein antibodies to the corresponding cytokines are immobilized.

T cell migration can be mentioned as the fourth novel function of ebastine and carebastine. In the initial stage of the onset of inflammation in an allergic disease, T cells permeate through vascular endothelial cells and such, and accumulate at inflammatory sites. The aforementioned Th2 type cytokines, inflammatory cytokines, and such are then released from the accumulated T cells to induce inflammation. Ebastine and carebastine inhibit transendothelial T cell migration in the initial stage of allergic immune response. The ebastine concentration required to inhibit T cell migration in an *in vitro* system is 1 µM to 10 µM, preferably 5 µM. In addition, the carebastine concentration required to inhibit T cell migration in an *in vitro* system is 1 µM to 200 µM, preferably 100 µM to 200 µM. The migration of T cells can be specifically measured by the method described in Example 5. Put simply, T cells are placed on one side of a layer of vascular endothelial cells and then the number of T cells that have moved to the other side is counted after a predetermined time.

The concentrations of ebastine and carebastine required to suppress the various allergic immune responses described above are those that exhibit activity *in vitro.* However, the concentrations used *in vivo* (in animals and the like) and treating human diseases can be readily determined by those skilled in the art using the aforementioned *in vitro* concentrations as a standard or reference, and using the analytical methods mentioned above or presented in the

### Examples.

As described above, ebastine and carebastine can be used to suppress allergic immune response, such as T cell proliferation, Th2 type cytokine production, inflammatory cytokine production by T cells and macrophages, and T cell migration, as well as inflammatory reactions arising from allergic immune response. Therefore, ebastine and carebastine can be used as agents to treat diseases involving these allergic immune responses.

Examples of such diseases include allergic rhinitis, bronchial asthma, atopic dermatitis, systemic lupus erythematosus, myasthenia gravis, chronic GVHD, and Th2 type autoimmune diseases (e.g., lupus nephritis).

In diseases like bronchial asthma, T cells have been found to migrate and accumulate at inflammatory sites to produce Th2 type cytokines at these sites. As described above, ebastine and carebastine suppress T cell migration and further suppress Th2 type cytokine production. Therefore, ebastine and carebastine are applicable as pharmaceuticals for the treatment of asthma and other diseases that involve such Th2 type cytokines.

Furthermore, in inflammatory diseases such as atopic diseases, inflammatory cytokines are detected at inflammatory sites. Ebastine and carebastine can suppress the production of inflammatory cytokines from both the macrophages and the T cells. Therefore, ebastine and carebastine can be effectively used as agents for the treatment of allergic inflammatory diseases that involve inflammatory cytokines, such as atopic diseases. Moreover, TNF-α and IL-6 antibody treatment is said to be effective in treating chronic rheumatoid arthritis and the inflammatory bowel disease, Crohn's disease, and the like. Hence, ebastine and carebastine may be used as agents to treat these diseases either alone or in combination with antibody treatment.

Furthermore, the present invention is based on the discovery of novel functions of the compound of formula (2) or salts thereof, and relates to the use of this compound as an agent other than an H1 blocker that suppresses allergic immune response involving immunocytes. In addition, the present invention relates to the use of the compound of formula (2) or salts thereof in methods of treating diseases associated with allergic immune response involving immunocytes, wherein the methods comprise administering the compound of formula (2) or salts thereof, or in the manufacture of agents that suppress allergic immune response involving immunocytes. The salts according to the present invention preferably include hydrochloride (epinastine hydrochloride) ; however, it is in no way limited thereto.

Examples of the novel functions of the compound of formula (2) or salts thereof include: suppression of T cell proliferation, suppression of Th2 type cytokine production, and suppression of Th1 type cytokine production. Namely, the present invention relates to the use of the compound of formula (2) or salts thereof as an agent that suppresses the aforementioned various allergic immune response induced by T cells that play a central role in allergic immune responses. Each of the function is described in detail below.

The first novel function of the compound of formula (2) or salts thereof includes the suppression of T cell proliferation. The *in vitro* optimal concentration required to suppress T cell proliferation can be determined based either on or by referring to the concentration of epinastine hydrochloride. The *in vitro* epinastine hydrochloride required to suppress T cell proliferation is 10 µM to 200 µM, preferably 20 µM to 200 µM.

As the second novel function of the compound of formula (2) or salts thereof, the ability to suppress the production of Th2 type cytokines can be mentioned. The *in vitro* optimal concentration required for the suppression of Th2 type cytokine production can be determined based either on or by referring to the concentration of epinastine hydrochloride. The *in vitro* epinastine hydrochloride concentration required to suppress the production of Th2 type cytokines is 10 µM to 200 µM, preferably 20 µM to 200 µM, and even more preferably 100 µM to 200 µM.

The ability to suppress the production of Th1 type cytokines can be exemplified as the third novel function of the compound of formula (2) or salts thereof. Th1 type cytokines are produced from T helper cell type 1, and are involved in cellular immunity and the symptoms of organ-specific autoimmune diseases (e.g., autoimmune thyropathy, multiple sclerosis, etc.) and inflammatory diseases (e.g., Crohn's disease, inflammatory bowel disease, rheumatoid arthritis, etc.). Psoriasis is also a Th1 type cytokine disease. Examples of Th1 type cytokines include IL-2 and IFN-γ. The identification and quantification of these cytokines can be performed using ELISA wherein antibodies against the cytokines are immobilized. The optimal in vitro concentration required for the suppression of Th1 type cytokine production can be determined based either on or by referring to the concentration of epinastine hydrochloride. The in vitro epinastine hydrochloride concentration required to suppress the production of Th1 type cytokines is 10 µM to 200 µM, preferably 20 µM to 200 µM, and even more preferably 100 µM to 200 µM.

The optimal concentrations of the compound of formula (2) or salts thereof required to suppress the various allergic immune responses described above used *in vivo* (in individuals such as animals) and those used for treating human diseases can be readily determined by one skilled in the art using the aforementioned concentrations in an in vitro system as standard or reference, and using the analytical methods presented in the Examples.

As described above, the compound of formula (2) or salts thereof can be used to suppress allergic immune responses, such as T cell proliferation, Th2 type cytokine production, and Th1 type cytokine production, as well as inflammatory reactions arising from the allergic immune response. Therefore, epinastine hydrochloride can be used as an agent to treat diseases involving these allergic immune responses.

Examples of these diseases include allergic rhinitis, bronchial asthma, atopic dermatitis, psoriasis vulgaris, autoimmune diseases involving imbalance of Th1/Th2 cytokines (e.g., systemic lupus erythematosus, rheumatic arthritis, etc.), Crohn's disease, inflammatory bowel disease, and multiple sclerosis.

Allergic immune response suppressing agents comprising, as active ingredient, ebastine, carebastine, or the compound of formula (2) or salts thereof may be used together with additives such as an excipient. Ebastine, carebastine, and the compound of formula (2) or salts thereof may be administered either orally or parenterally. Examples of dosage forms include tablets, capsules, granules, powders, and injections. When administered orally as tablets, capsules, granules, powders or such, the following may be added as needed: excipients such as lactose, crystalline cellulose, starch, and vegetable oil; lubricants such as magnesium stearate and talc; binders such as hydroxypropyl cellulose and polyvinylpyrrolidone; disintegrators such as carboxymethylcellulose calcium and low-substituted hydroxypropyl methylcellulose; coatings such as hydroxypropyl methylcellulose, Macrogol, and silicone resin; and film-forming agents such as gelatin membranes. The formulation of these agents can be performed according to conventional methods ordinary used by those skilled in the art. In addition, ebastine, carebastine, and the compound of formula (2) or salts thereof may also be incorporated into a drug delivery system or the like, if needed.

### Brief Description of the Drawings

FIG. 1 shows the results of analyzing the effects of ebastine on T cell proliferation due to different stimulations. The reproducibility of these data was confirmed by five independent analyses using samples derived from different donors.
FIG. 2 shows the results of analyzing the effects of carebastine (A) and epinastine hydrochloride (B) on T cell proliferation due to different stimuli.
FIG. 3 shows the results of analyzing the effects of cetirizine hydrochloride (A) and ketotifen fumarate (B) on T cell proliferation due to different stimuli.
FIG. 4 shows the effects of ebastine on the production of IL-2 (A) and IFN-γ (B) from peripheral blood T cells upon application of different stimuli. The reproducibility of these data was confirmed by five independent analyses using samples derived from different donors.
FIG. 5 shows the effects of carebastine on the production of IL-2 (A) and IFN-γ (B) from peripheral blood T cells upon application of different stimuli.
FIG. 6 shows the effects of epinastine hydrochloride on the production of IL-2 (A) and IFN-γ (B) from peripheral blood T cells upon application of different stimuli.
FIG. 7 shows the effects of cetirizine hydrochloride on the production of IL-2 (A) and IFN-γ (B) from peripheral blood T cells upon application of different stimuli.
FIG. 8 shows the effects of ketotifen fumarate on the production of IL-2 (A) and IFN-γ (B) from peripheral blood T cells upon application of different stimuli.
FIG. 9 shows the effects of ebastine on the production of IL-4 (A) and IL-5 (B) from peripheral blood T cells upon application of different stimuli. The reproducibility of these data was confirmed by five independent analyses using samples derived from different donors.
FIG. 10 shows the effects of carebastine on the production of IL-4 (A) and IL-5 (B) from peripheral blood T cells upon application of different stimuli.
FIG. 11 shows the effects of epinastine hydrochloride on the production of IL-4 (A) and IL-5 (B) from peripheral blood T cells upon application of different stimuli.
FIG. 12 shows the effects of cetirizine hydrochloride on the production of IL-4 (A) and IL-5 (B) from peripheral blood T cells upon application of different stimuli.
FIG. 13 shows the effects of ketotifen fumarate on the production of IL-4 (A) and IL-5 (B) from peripheral blood T cells upon application of different stimuli.
FIG. 14 shows the effects of ebastine on the production of IL-6 (A) and TNF-α (B) from peripheral blood T cells upon application of different stimuli. The reproducibility of these data was confirmed by five independent analyses using samples derived from different donors.
FIG. 15 shows the effects of carebastine on the production of IL-6 (A) and TNF-α (B) from peripheral blood T cells upon application of different stimuli.
FIG. 16 shows the effects of epinastine hydrochloride on the production of IL-6 (A) and TNF-α (B) from peripheral blood T cells upon application of different stimuli.
FIG. 17 shows the effects of cetirizine hydrochloride on the production of IL-6 (A) and TNF-α (B) from peripheral blood T cells upon application of different stimuli.
FIG. 18 shows the effects of ketotifen fumarate on the production of IL-6 (A) and TNF-α (B) from peripheral blood T cells upon application of different stimuli.
FIG. 19 shows the inhibitory effects of ebastine (A) and carebastine (B) on T cell migration through the vascular endothelial cells due to PHA activation. The reproducibility of (A) was confirmed by three independent analyses using samples derived from different donors.
FIG. 20 shows the effects of epinastine hydrochloride (A), cetirizine hydrochloride (B), and ketotifen fumarate (C) on T cell migration through the vascular endothelial cells due to PHA activation.

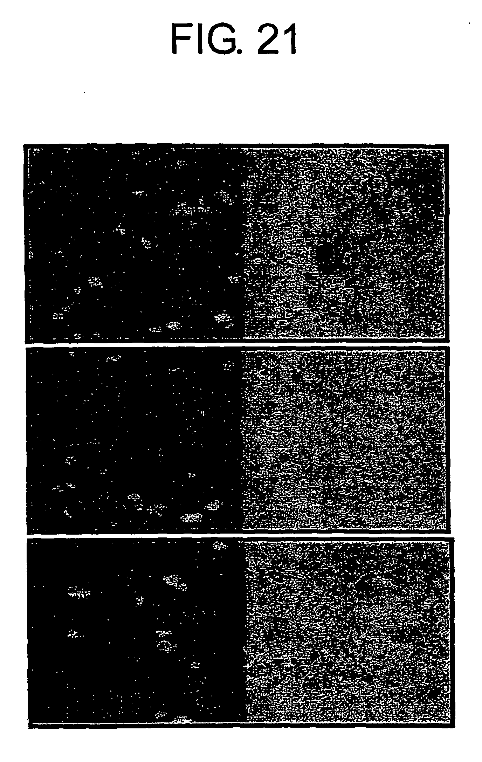

FIG. 21 shows photographs indicating the effects of ebastine on the adhesion of PHA-stimulated T cells to endothelial cells. Treatments were performed with 0 µM, 1.0 µM, and 5.0 µM ebastine, from the top to bottom panels, respectively.
FIG. 22 shows the effects of ebastine on the production of IL-6 (A) and TNF-α (B) from macrophages upon stimulation with LPS. The reproducibility of these data was confirmed by five independent analyses using samples derived from different donors.
FIG. 23 shows the effects of carebastine on the production of IL-6 (A) and TNF-α (B) from macrophages upon stimulation with LPS.
FIG. 24 shows the effect of epinastine hydrochloride on the production of IL-6 (A) and TNF-α (B) from macrophages upon stimulation with LPS.
FIG. 25 shows the effects of cetirizine hydrochloride on the production of IL-6 (A) and TNF-α (B) from macrophages upon stimulation with LPS.
FIG. 26 shows the effects of ketotifen fumarate on the production of IL-6 (A) and TNF-α (B) from macrophages upon stimulation with LPS.
FIG. 27 shows photographs indicating the effects of ebastine on Cas-L and FAK tyrosine phosphorylations in PHA-stimulated T cells.

### Best Mode for Carrying Out the Invention

Herein below, the present invention will be specifically described using Examples; however, it is not to be construed as being limited thereto.

### [Example 1] Effects of H1 Blockers on T Cell Proliferation via Different Activation Pathways

All H1 blockers used for this comparative study belong to the long-acting second-generation H1 blockers recently used in Japanese clinical practice. Specifically, these included ebastine (trade name Ebastel), its active metabolite carebastine, epinastine hydrochloride (Alesion), and cetirizine hydrochloride (Zyrtec), all confirmed to be effective as a single dose per day to improve compliance by patients. As a control, ketotifen fumarate (Zaditen) which is said to have a high selectivity and affinity to H1 receptors among conventional H1 blockers was added, and in total, five types of H1 blockers were examined.

The maximum blood concentration of ebastine is 0.03 µM, and that of carebastine, the active metabolite of ebastine, is 0.1 µM. Epinastine hydrochloride, cetirizine hydrochloride, and ketotifen fumarate act as the same structure even after absorption via the intestinal tract, and their maximum blood concentrations are 0.1 µM, 0.5 µM, and 0.01 µM, respectively. The present experiments were performed using following concentrations: ebastine (0.1 µM to 10.0 µM), carebastine (1.0 µM to 100. 0 µM), epinastine hydrochloride (1.0 µM to 100.0 µM), cetirizine hydrochloride (5.0 µM to 500.0 µM), and ketotifen fumarate (0.1 µM to 10.0 µM).

In order to analyze the effects of H1 blockers on T cell proliferation, three different costimulation pathways were used. The first pathway was PMA, the second pathway stimulation on CD28 that is one of the representative T cell costimulation molecules, and the third pathway stimulation on CD26 that is expressed on CD4⁺ memory T cells. The simulation on CD3 was applied together with all of these stimulations. The stimulation on CD3, CD26, and CD28 was performed using monoclonal antibodies OKT3, 1F7 (Morimoto C *et al*., 1989, *supra*), and 4B10 (immi CD *et al*., 1991, *supra*), respectively. OKT3 was purchased from the American Tissue Culture Collection (ATCC, Rockville, MD), while 4B10 and 1F7 were purified from mouse ascites of hybridomas established in the present inventors' laboratory (Gimmi CD *et al*., 1991, *supra;* Morimoto C *et al*., 1989, *supra*).

Specifically, human peripheral blood mononuclear cells (PBMC) were isolated, from the peripheral blood of normal healthy persons by specific gravity centrifugation using Ficoll/Paque (Pharmacia Biotech Inc., Piscataway, NJ). Unfractionated mononuclear cells were separated using the E-rosette positive (E⁺) method and used as unstimulated T cells. The mononuclear cells were adhered to a plastic plate for 24 hours at 37°C, and then removed by culturing for one hour in 5 mM L-leucine methyl ester HCL (Sigma Chemical Co.) solution. A portion of the recovered cells was stained with anti-CD3 antibodies and anti-CD14 antibodies, and the purity of the cells was confirmed by flow cytometry. As a result, 97% or more of the cells were found to be CD3 positive, while 1% or less of the cells were CD14 positive. The cells obtained with the aforementioned purity were used as T cells.

Phosphate-buffer saline (PBS) (100 µl) containing either one of the monoclonal antibodies, OKT3 (0.05 µg/ml), 1F7 (1 µg/ml), or 4B10 (2 µg/ml), was coated in triplicate according to a known method (Dang NH *et al*., 1990, supra) on flat-bottomed 96-well plates (Coster, Cambridge, MA), and the plates were then incubated overnight at 4°C. Before use, these plates were washed once with PBS (200 µl). The highly purified T cells (1x 10⁵) described above were resuspended in RPMI medium (200 µl) containing 10% human AB serum and ebastine of four different concentrations (0 µM, 1 µM, 5 µM, and 10 µM). In order to evaluate PMA stimulation, a cell suspension containing PMA (5 ng/ml) was poured into the wells coated with OKT3. The cells were cultured for three days under wet conditions at 37°C and 5% CO₂. The cells were stimulated for eight hours with 1 µCi ³H-thymidine (ICN Radiochemicals, Irvine, CA) per well, recovered on a glass fiber filter (Wallac, Turku, Finland) to quantify the incorporated radioactivity with a liquid scintillation counter (Wallac).

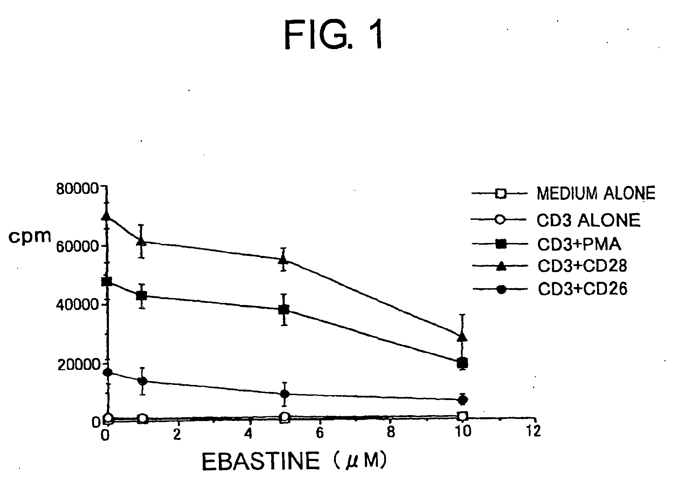

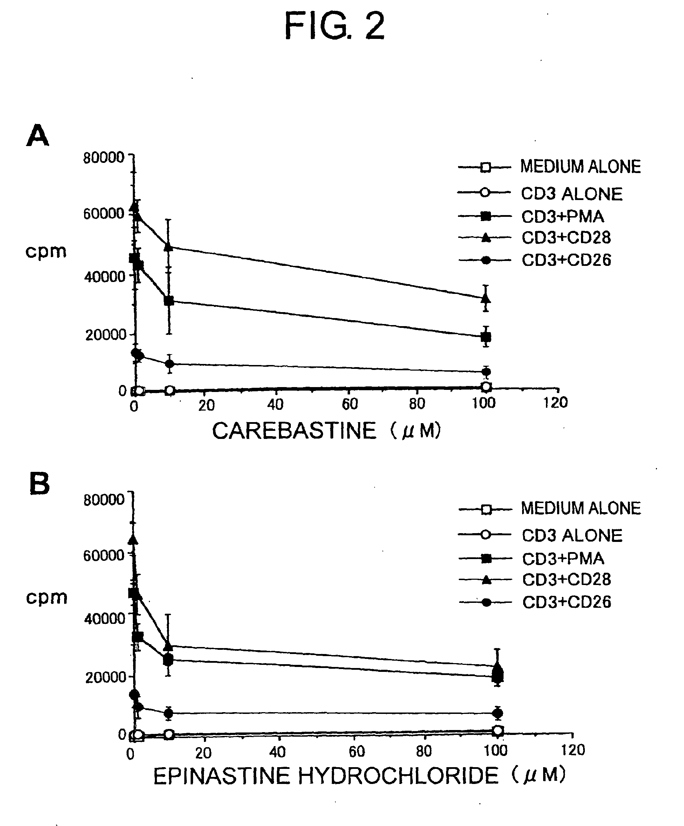

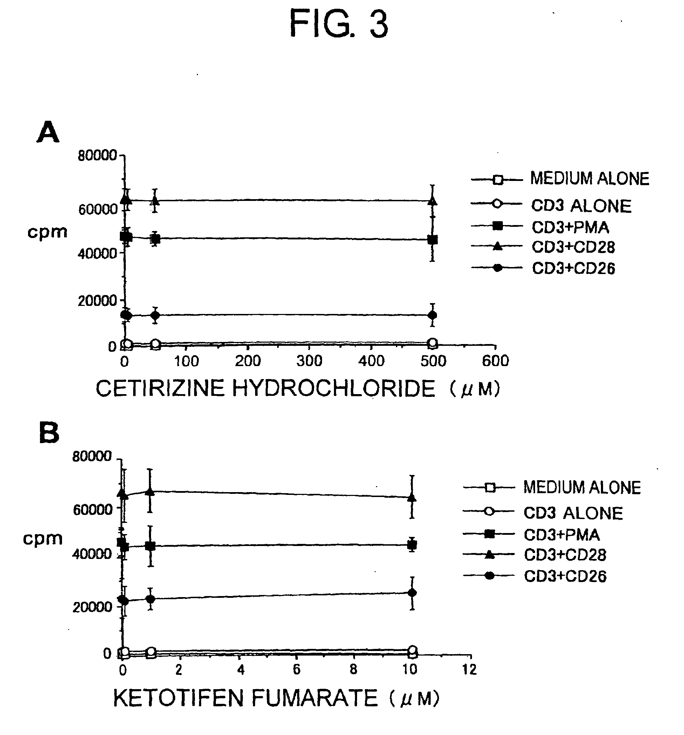

As shown in FIGs. 1 to 3, the level of inducing T cell proliferation with the stimulation on CD3 alone was low. Conspicuous T cell proliferation was observed when the stimulation on CD3 was combined with additional second stimulation on CD26, CD28, or PMA. Under these conditions, ebastine clearly inhibited T cell proliferation in a concentration-dependent manner. Under each of the costimulation conditions and at all of the concentrations used in this Example, cetirizine hydrochloride and ketotifen fumarate hardly inhibited T cell proliferation (FIG. 3). On the other hand, ebastine (1 µM, 5 µM, and 10 µM), carebastine (1 µM, 10 µM, and 100 µM), and epinastine hydrochloride (1 µM, 10 µM, and 100 µM) inhibited T cell proliferation by 10% to 50% (FIGs. 1 and 2) under each of the costimulation conditions. T cell proliferation induced by the anti-CD26 antibody tended to exhibit a slight resistance to the inhibition by ebastine. Moreover, at ebastine concentrations of 20 µM to 50 µM, nearly 100% of T cell proliferation was inhibited in a certain donor.

The inventors analyzed whether or not the above results were due to the side effect of cell toxicity of ebastine, carebastine, and epinastine hydrochloride by evaluating the cell survival rate through a trypan blue exclusion test. The T cell survival rate was 95% or higher at each measurement point in all experiments. This indicates that the inhibitory effect of ebastine, carebastine, and epinastine hydrochloride was not due to their side effect of cell toxicity.

### [Example 2] Effects of H1 Blockers on Th1 type Cytokine Production

In order to analyze whether or not H1 blockers have an inhibitory effect on cytokine production induced by the various costimulations described above, the present inventors measured Th1 type cytokines (IL-2, and IFN-γ) in the culture supernatant induced by the various costimulations described above.

The antibody-coated plates and T cells were prepared similarly as in Example 1 except that the OKT3 concentration was 0.5 µg/ml. The cytokine production by T cells was analyzed in triplicate using 96-well flat-bottomed plates by the same method employed in the T cell proliferation analysis described above. After culturing for 24 hours, the culture supernatant was tested using ELISA kit (IL-2: Biosource International, Camarillo, CA; IFN-γ: R&D systems, Minneapolis, MN) to quantify IL-2 and IFN-γ.

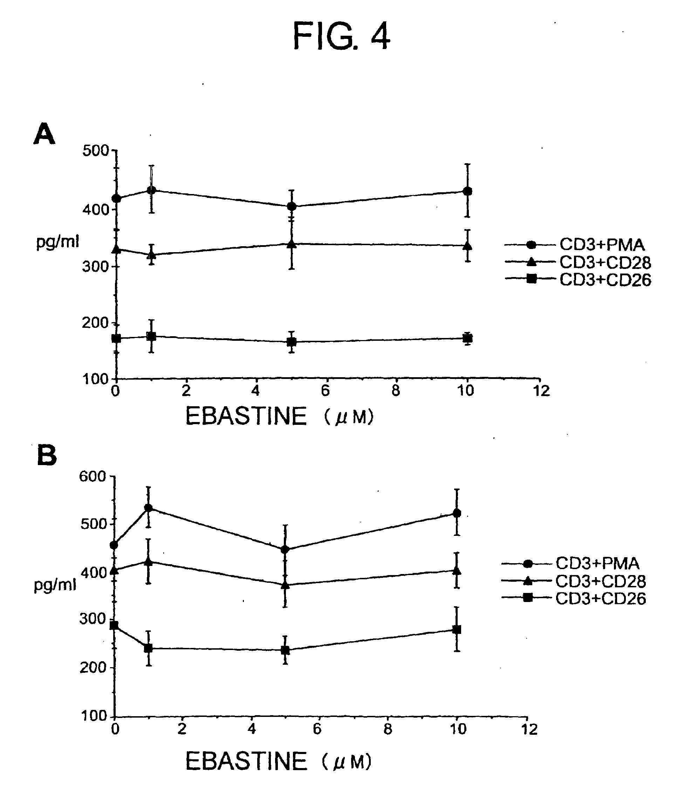

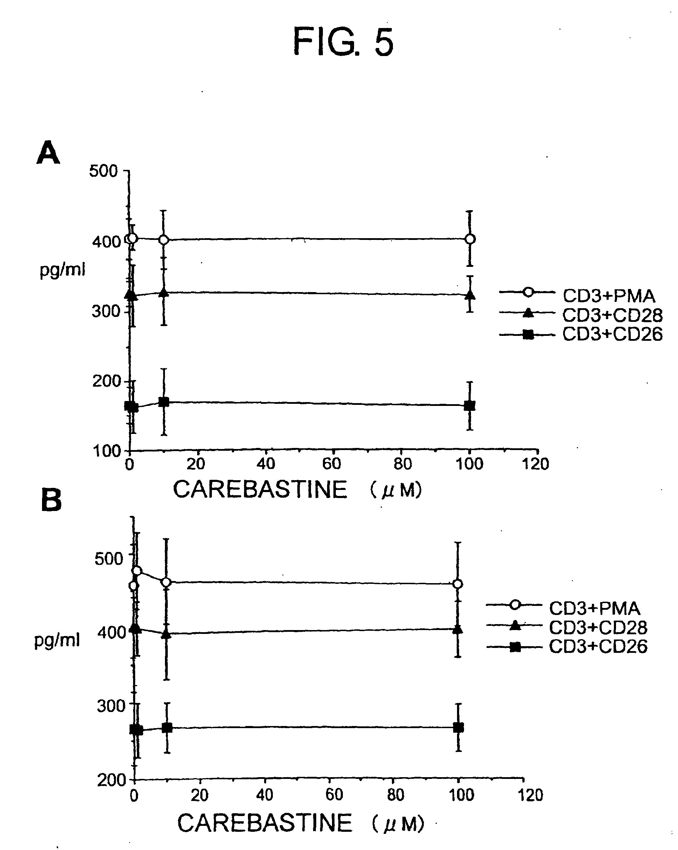

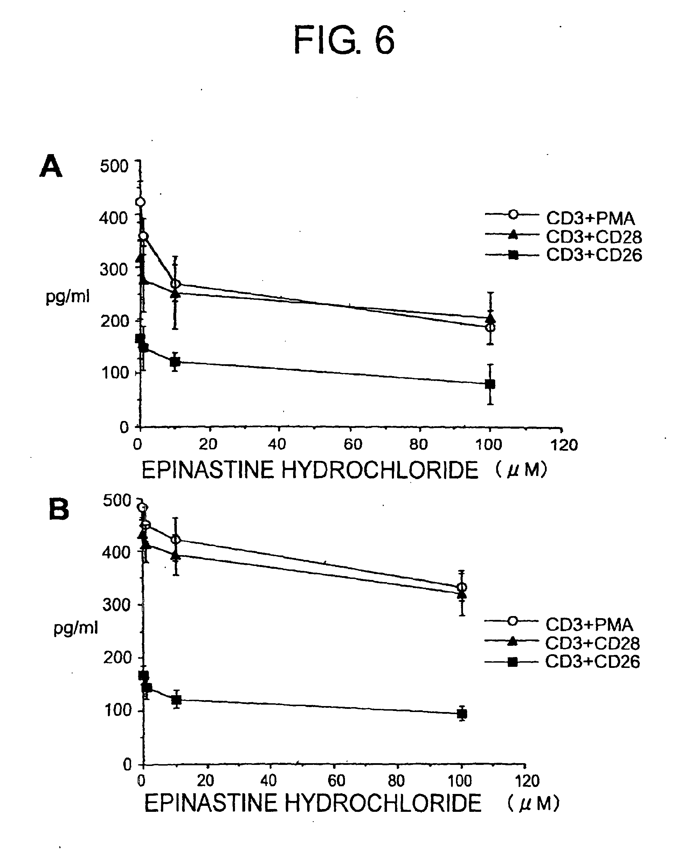

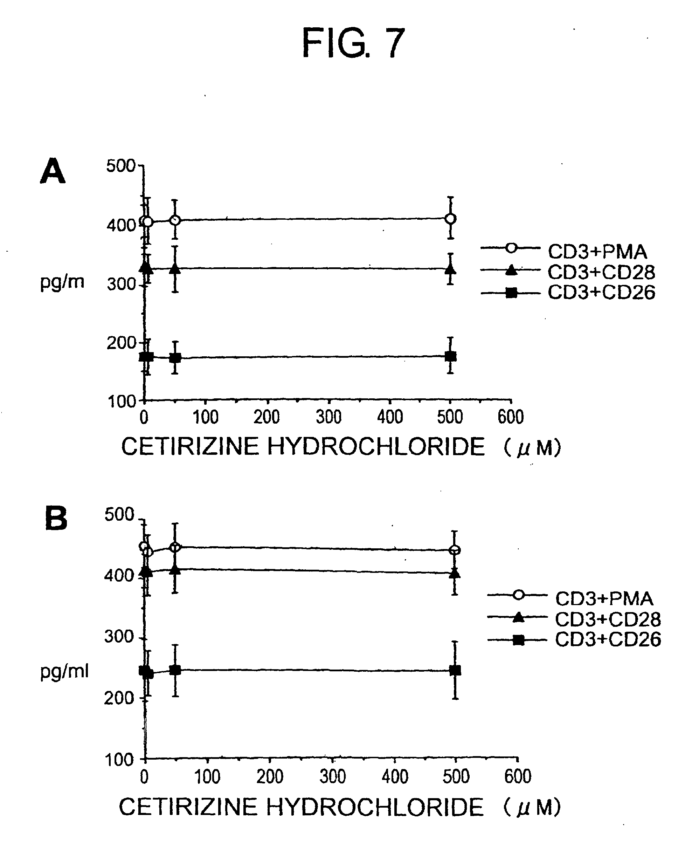

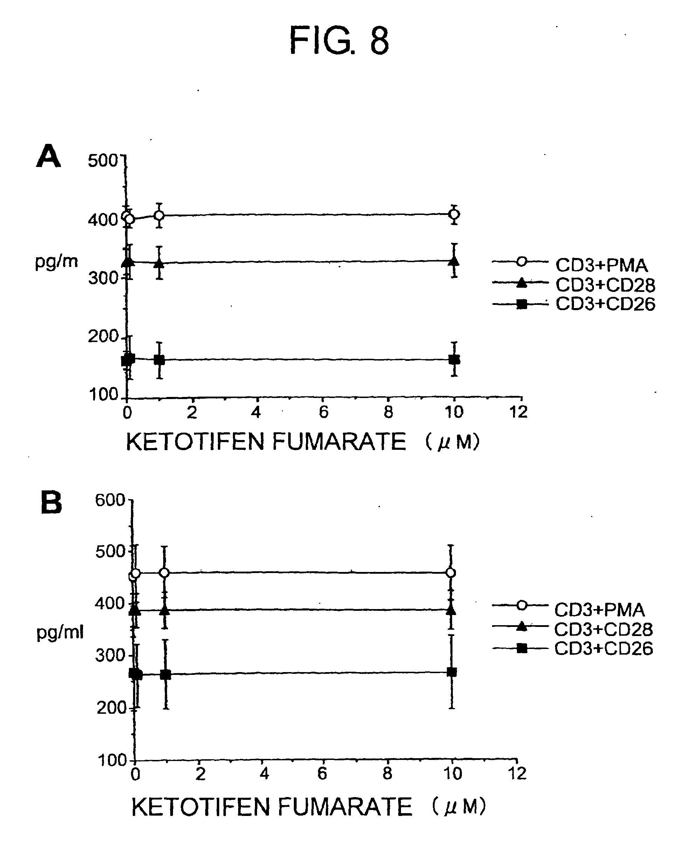

As shown in FIGs. 4A and 5A, ebastine and carebastine did not inhibit IL-2 production under any of the costimulation conditions even at the maximum concentrations (10 µM for ebastine, and 100 µM for carebastine). In addition to IL-2, ebastine and carebastine did not have a clear effect on IFN-γ production at any of the concentrations of 0.1 µM to 10 µM and 1 µM to 100 µM, respectively (FIGs. 4B and 5B). On the other hand, epinastine hydrochloride suppressed both IL-2 and IFN-γ production due to each costimulation in a dose-dependent manner (FIG. 6). In addition, cetirizine hydrochloride and ketotifen fumarate did not have a clear effect on IL-2 and IFN-γ production due to each costimulation (FIGs. 7 and 8). Therefore, these results indicated that ebastine and carebastine do not inhibit Th1 type cytokine production under any costimulation conditions.

### [Example 3] Effects of H1 Blockers on Th2 type Cytokine Production

Th2 type CD4 T cells play an important role in allergic diseases. Therefore, the present inventors analyzed the effects of ebastine on Th2 type cytokines (IL-4, and IL-5) induced by the various costimulations described above. This analysis was performed similarly as in Example 2 except that ELISA (IL-4: Biosource International, Camarillo, CA; IL-5: R&D systems, Minneapolis, MN) was used to quantify IL-4 and IL-5.

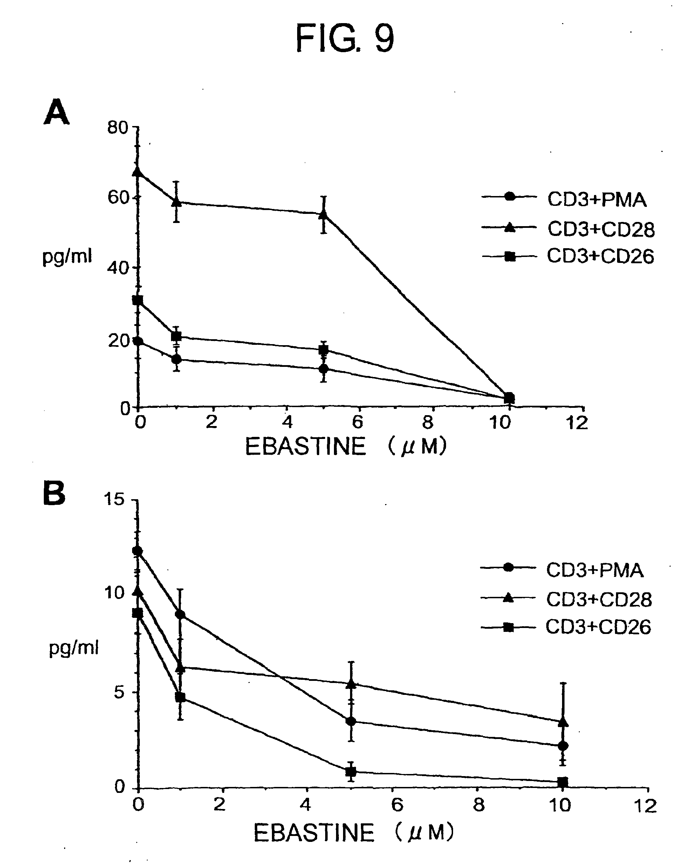

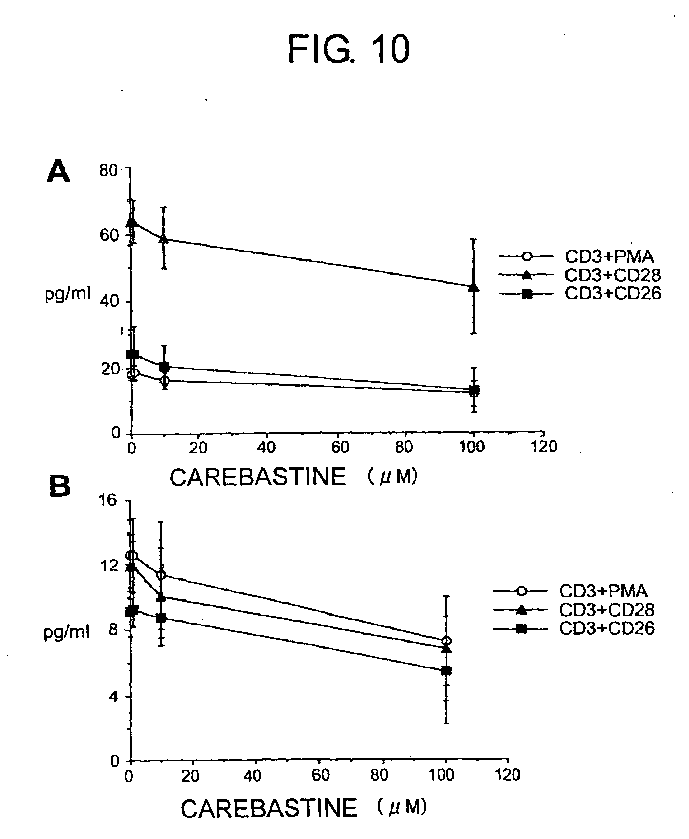

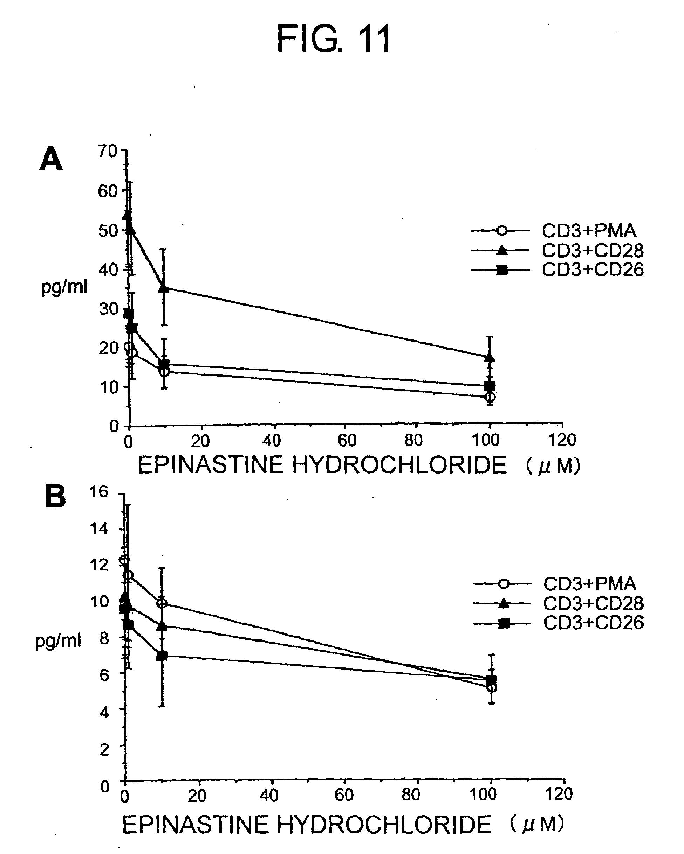

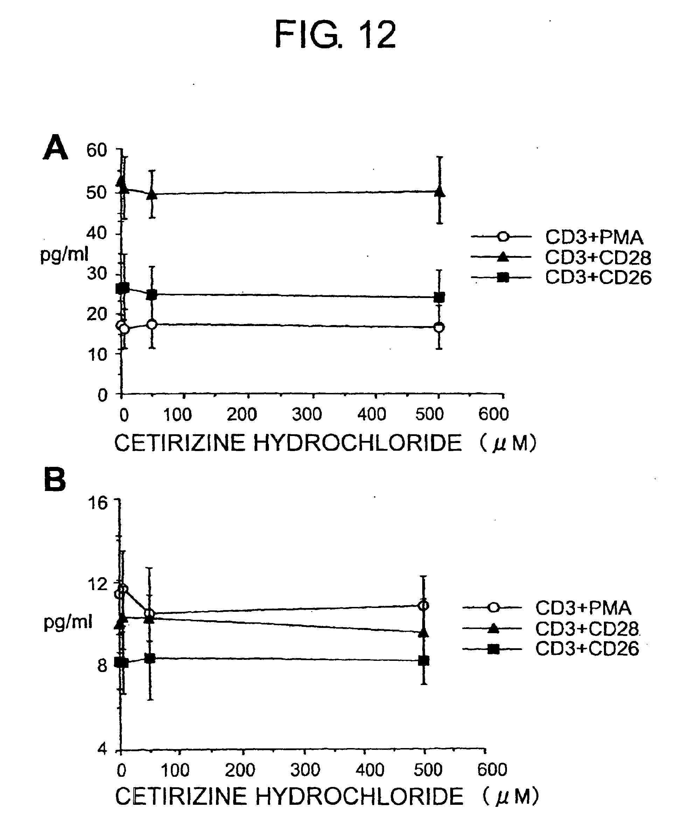

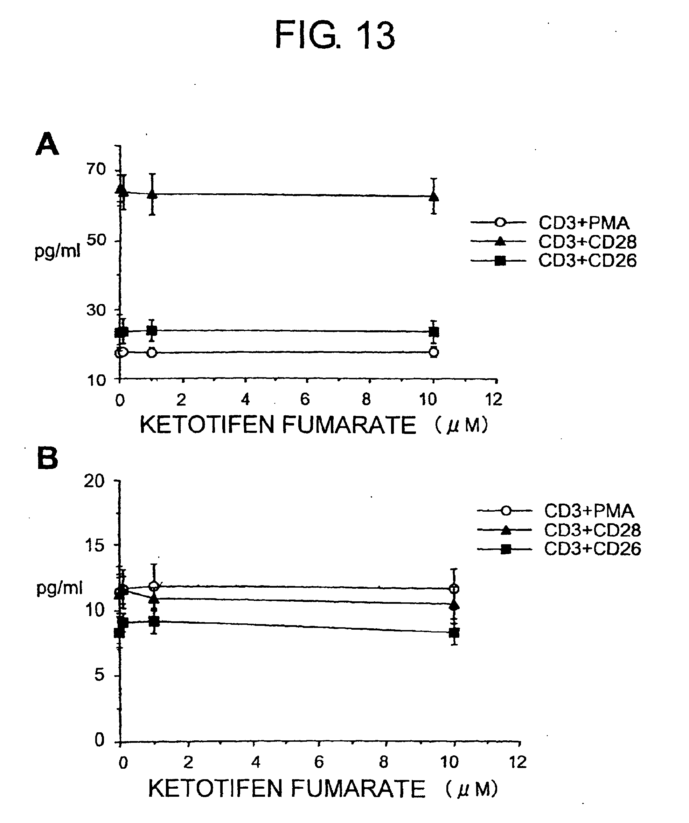

As shown in FIGs. 9A, 10A, and 11A, ebastine, carebastine, and epinastine hydrochloride inhibited IL-4 production under each costimulation condition in a concentration-dependent manner. In particular, ebastine nearly completely inhibited IL-4 production from T cells at 10 µM. Furthermore, as shown in FIGs. 9B, 10B, and 11B, ebastine, carebastine, and epinastine hydrochloride inhibited IL-5 production in a concentration-dependent manner under each of the costimulation conditions. In comparison to the effect of ebastine on IL-4 production from T cells, IL-5 production was inhibited by 50% or more at an ebastine concentration of 5 µM, indicating IL-5 production being more sensitive to ebastine. This result was reproduced in five independent donors. Moreover, at a concentration of 20 µM, IL-4 and IL-5 productions were 100% suppressed in all donors. In addition, in the suppression of cytokine production, the carebastine dose was higher than that of ebastine. On the other hand, both cetirizine hydrochloride and ketotifen fumarate did not have a clear effect on IL-4 and IL-5 productions due to each costimulation (FIGs. 12 and 13).

### [Example 4] Effects of H1 Blockers on Inflammatory Cytokine Production

The present inventors analyzed the effects of H1 blockers on the production of inflammatory cytokines, such as IL-6 and TNF-α, from T cells due to the various costimulation pathways described above. This analysis was performed similarly as in Example 2 above except for the use of an ELISA kit for measuring IL-6 and TNF-α (R&D systems, Minneapolis, MN).

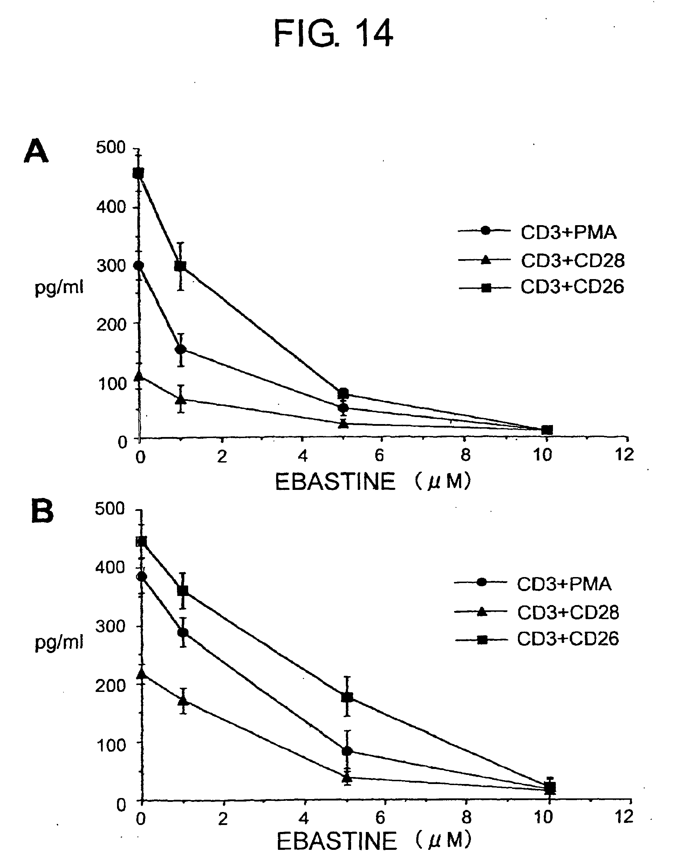

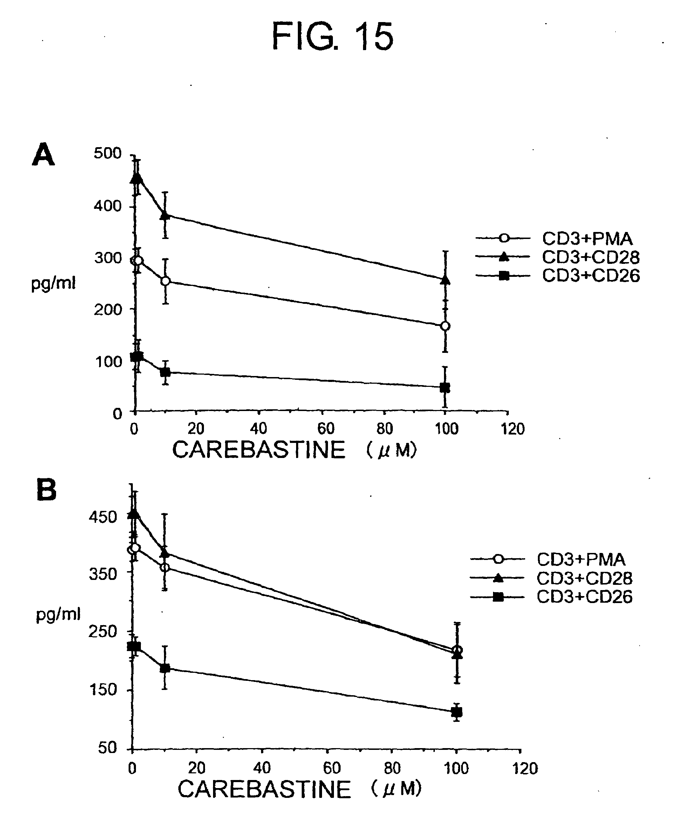

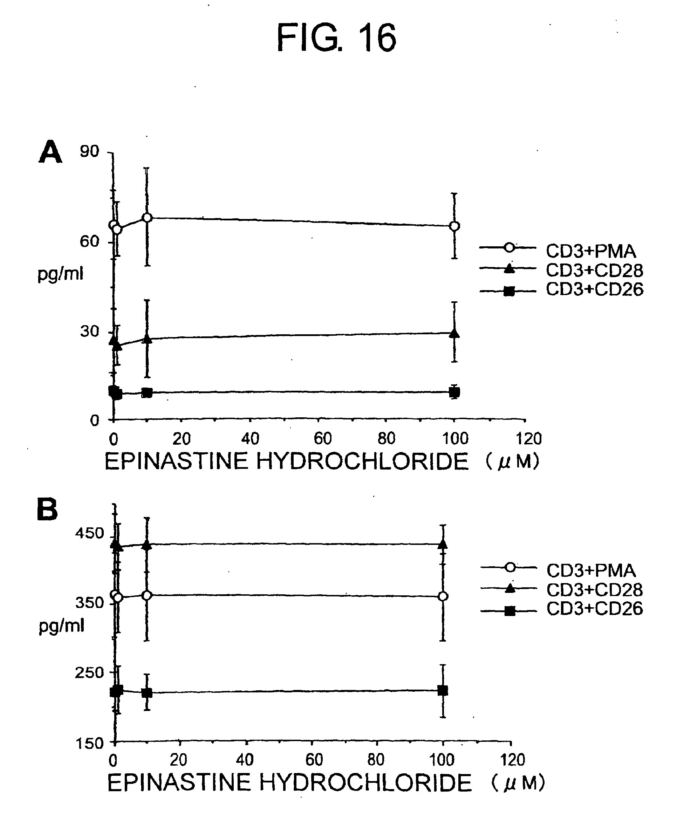

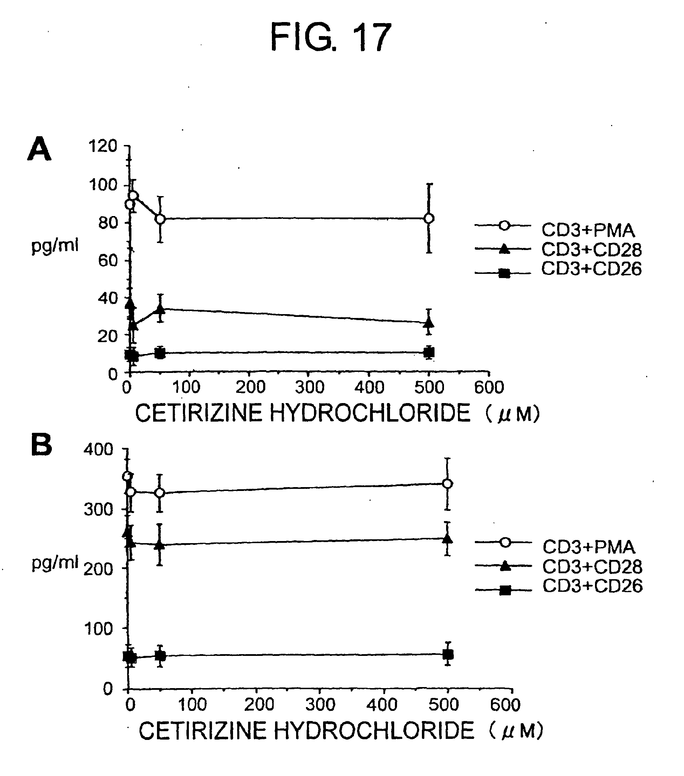

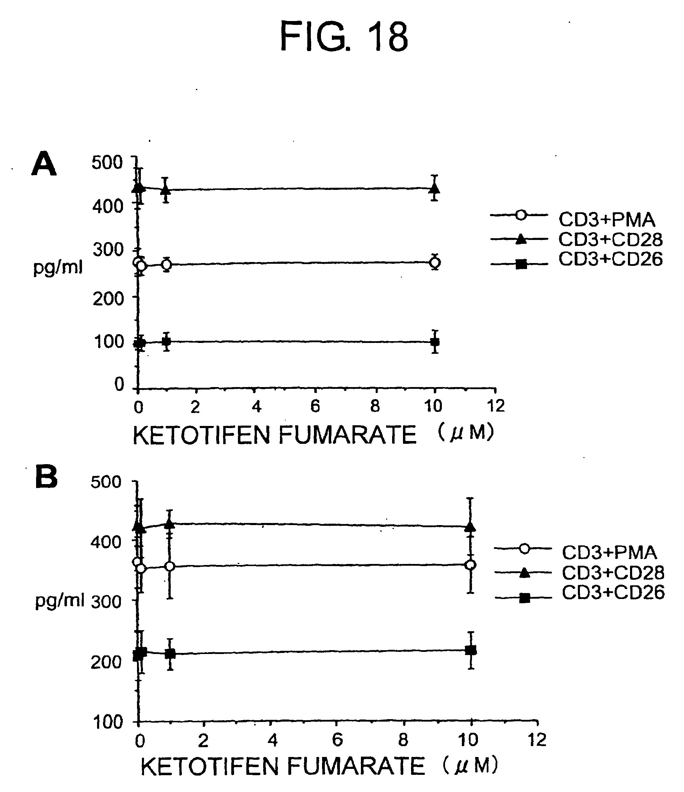

As shown in FIGs. 14 and 15, ebastine and carebastine inhibited, in a concentration-dependent manner, the inflammatory cytokines IL-6 and TNF-α from T cells under each of the costimulation conditions. Ebastine achieved 100% suppression at concentrations from 10 µM to 20 µM. This result was reproduced in three independent donors. On the other hand, regarding epinastine hydrochloride, no clear effect of suppressing IL-6 and TNF-α production was found by any of the costimulations (FIG. 16). In addition, cetirizine hydrochloride and ketotifen fumarate did not have a clear effect on IL-6 and TNF-α productions due to each costimulation (FIGs. 17 and 18).

### [Example 5] Effects of H1 Blockers on Transendothelial T Cell Migration

In order to determine the effect of ebastine on T cell permeation, the present inventors analyzed vascular transendothelial migration of PHA-stimulated T cells in vitro. Since fresh resting-stage T cells hardly migrate by permeating a monolayer of endothelial cells, these T cells were activated using PHA (10 µg/ml) prior to performing transendothelial migration analysis.

Specifically, the analysis of transendothelial migration was performed by slightly modifying a known method (Iwata S, Yamaguchi N, Munakata Y, Ikushima H, Lee JF, Hosono O, Schlossman SF, Morimoto C. "CD26/DPPIV differentially regulates the chemotaxis of T cells and monocytes toward RANTES. Possible mechanism for the switch from innate to acquired immune response." Int Immunol. 11:417-426, 1999). Human endothelial cell strain ECV304 was obtained from the American Tissue Culture Collection (ATCC; Rockville, MD) and cultured for 48 hours on Transwell cell culture inserts (Corning Costar, Cambridge, MA) with a pore size of 3.0 µm. The cells were loaded onto a 24-well plastic plate for cell culture (FALCON), and mixed with H1 blockers at various concentrations using Transwell assay medium (RPMI1640 medium, BSA 0.6%, Penicillin G/Streptomycin 1%, HEPES 50 mM). Two-hundred µl of PHA-stimulated T cells (1x 10⁷ /ml) were then placed in the upper chamber and 600 µl of assay medium was placed in the lower chamber, and migration was performed for eight hours at 37°C under 5% CO₂. After recovery of the cells, the number of cells was counted for one minute by flow cytometry (FACSCalibur, Nippon Becton-Dickinson, Tokyo, Japan).

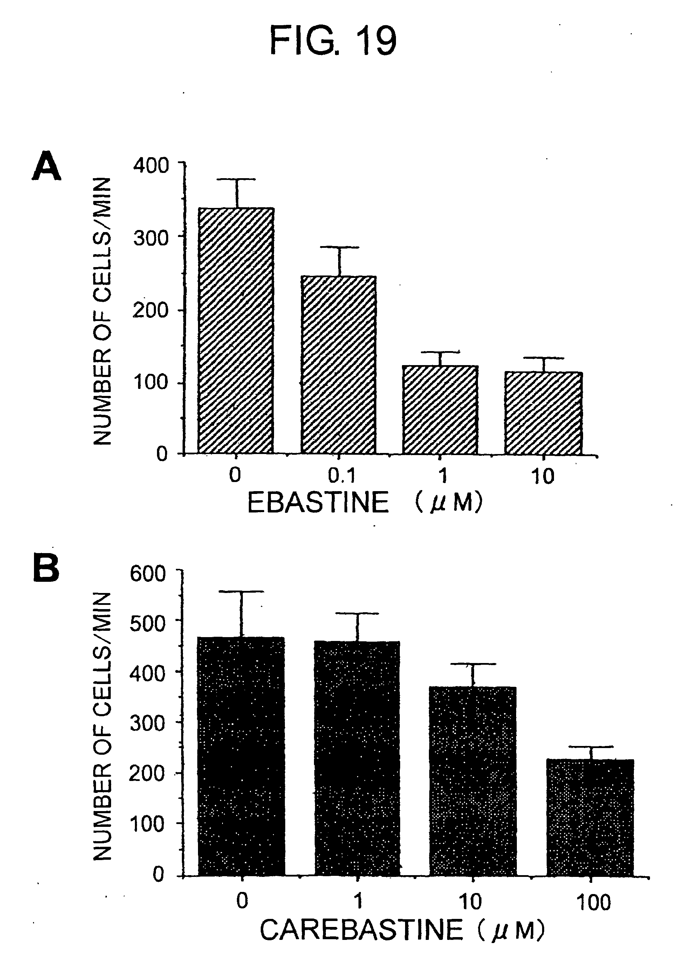

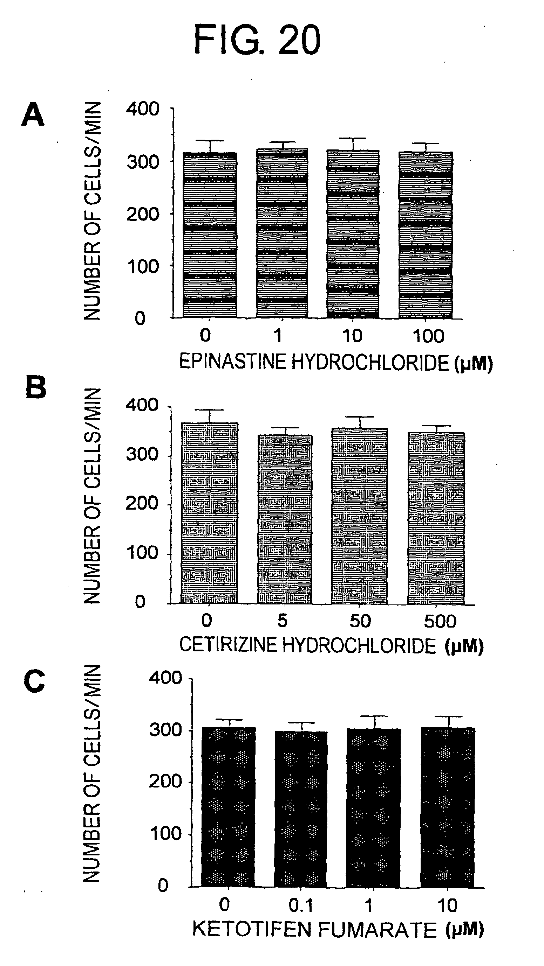

As shown in FIG. 19A, T cell migration was markedly inhibited in the presence of ebastine in a concentration-dependent manner. It is noteworthy that the inhibition of T cell migration reached a plateau level at a concentration of 5 µM. In addition, T cell migration was also suppressed in the presence of carebastine in a concentration-dependent manner (FIG. 19B). Conversely, when ECV cells were pretreated with ebastine of various concentrations for 48 hours, washed and then exposed to PHA-stimulated T cells, ebastine did not show effect on the T cell migration via ECV cells even at the maximum concentration used in the experiment (10 µM) (not shown) . Thus, it was shown that T cell migration is suppressed when ebastine is present at the time of T cell migration analysis. On the other hand, epinastine hydrochloride, cetirizine hydrochloride, and ketotifen fumarate did not have a clear effect on the migration of PHA-stimulated T cells (FIG. 20).

Ebastine suppressed PHA-stimulated T cell migration in a dose-dependent manner. Thus, the adhesion of PHA-stimulated T cells to endothelial cells was analyzed in order to elucidate the action mechanism of the suppression of T cell migration by ebastine. PHA-stimulate T cells were reacted for 30 minutes with the fluorescent dye 3'-O-Acetyl-2',7'-bis(carboxyethyl)-4-or5-carboxyfluorescein, diacetoxymethyl ester (BECEF-AM), treated with H1 blocker, ebastine (1.0 µM to 5.0 µM), and then plated on a culture dish to which endothelial cells were adhered. After six hours, the culture dish was washed three times with PBS. Because of observation under a fluorescent microscope, ebastine was found to suppress the adhesion of PHA-stimulated T cells to endothelial cells in a dose-dependent manner.

Moreover, cell surface staining experiments were performed using monoclonal antibodies against various adhesion molecules on T cells, such as CD11a, CD29, CD44, and CD47, activation antigens such as CD25 and CD26, and ligands of the adhesion molecules on endothelial cell membranes such as ICAM-1 and VCAM-1.

PHA-stimulated T cells and endothelial cells were cultured for 6 hours in the presence of ebastine (1.0 µM to 5.0 µM) at 37°C under 5% CO₂, and antibodies to various cell surface molecules (CD3, CD11a, CD25, CD26, CD29, CD44, CD47, ICAM-1, and VCAM-1) were reacted with the cells for 30 minutes at 4°C. The cells were washed with PBS containing 2% FBS and 0.02% sodium azide (Sigma), and further reacted with FITC-labeled goat anti-mouse IgG antibody (Sigma) for 30 minutes at 4°C. Washed three more times with PBS, and then the cell fluorescence intensity was measured by flow cytometry. The antibodies used to identify T cell surface molecules were: anti-CD3 antibody (OKT3), anti-CD11a antibody (BD PharMingen, NJ, USA), anti-CD25 antibody (BD PharMingen), anti-CD26 antibody (1F7), anti-CD29 antibody (4B4), anti-CD44 antibody (BD PharMingen), and anti-CD47 antibody (BD PharMingen) ; and those for the ligands of the adhesion molecules on the endothelial cell surfaces were: anti-ICAM-1 antibody and anti-VCAM-1 antibody. The results are presented in Tables 1 and 2.

**Table 1**

| **Ebastine concentration** | **0 µM** | **1 µM** | **5 µM** |
|---|---|---|---|
| **(-)** | 0.3 | 0.3 | 0.3 |
| **CD3** | 10.6±0.26 | 10.6±0.2: N.S. | 10.9±0.26: N.S. |
| **CD11a** | 53.1±0.44 | 53.8±0.44: N.S. | 53.9±0.4: N.S. |
| **CD25** | 18.2±0.56 | 18.3±0.35: N.S. | 16.5±0.26: ** |
| **CD26** | 102.0±0.69 | 99.1±0.36: ** | 87.8±0.36: *** |
| **CD29** | 28.0±0.56 | 25.0±0.26: * | 22.1±0.36: *** |
| **CD44** | 48.7±0.62 | 47.6±0.30: N.S. | 47.9±0.36: N.S. |
| **CD47** | 14.5±0.35 | 11.4±0.17: ** | 10.0±0.26: *** |

| | | | |
|---|---|---|---|
| *: P<0.05, | | | |
| **: P<0.01, | | | |
| ***: P<0.001, N.S.: Not Significant | | | |

**Table 2**

| Ebastine concentration | 0 µM | 1 µM | 5 µM |
|---|---|---|---|
| (-) | 0.3 | 0.3 | 0.3 |
| ICAM-1 | 20.3±0.41 | 20.7±0.52 | 20.4±0.26 |
| VCAM-1 | 0.3 | 0.3 | 0.3 |

After treatment with ebastine for eight hours, among all the adhesion molecules and activation antigens tested on the T cells, the expression of CD26, CD29, and CD47 was markedly effected (Table 1). At the ebastine concentration of 5 µM, the expression levels of CD26, CD29, and CD47 decreased by approximately 14%, 21%, and 31%, respectively. On the other hand, the expression levels of CD3, CD11a, CD25, and CD44 were completely unaffected (Table 1). In addition, ebastine had no effect on the fluorescence intensity of ligands, ICAM-1 and VCAM-1, of the adhesion molecules on the endothelial cell membrane (Table 2). Since CD26, CD29, and CD47 have been reported to be involved in cell migration, these results partially explain the inhibitory effect of ebastine on T cell migration.

### [Example 6] Effects of H1 Blockers on Inflammatory Cytokine Production by Macrophages

Macrophages participate in the early stages of the host-protection mechanism against infection and in the local regulation of immune/inflammatory reactions (Johnston RB Jr. "Monocytes and macrophages." N Eng J Med. 318:747-752, 1988). Therefore, the effects of ebastine on the production of inflammatory cytokines such as IL-6 and TNF-α were analyzed.

Specifically, in order to produce IL-6 and THF-α from macrophages, peripheral blood mononuclear cells were adhered to a plastic plate to isolate and recover macrophages. These macrophages (1x 10⁶/ml) were suspended in RPMI containing 10% FCS and stimulated with LPS (1 µg/ml). After culturing for eight hours at 37°C under 5% CO₂, the culture supernatant was recovered and the amount of produced IL-6 and TNF-α quantified using an ELISA kit for measuring IL-6 and TNF-α (R&D systems, Minneapolis, MN).

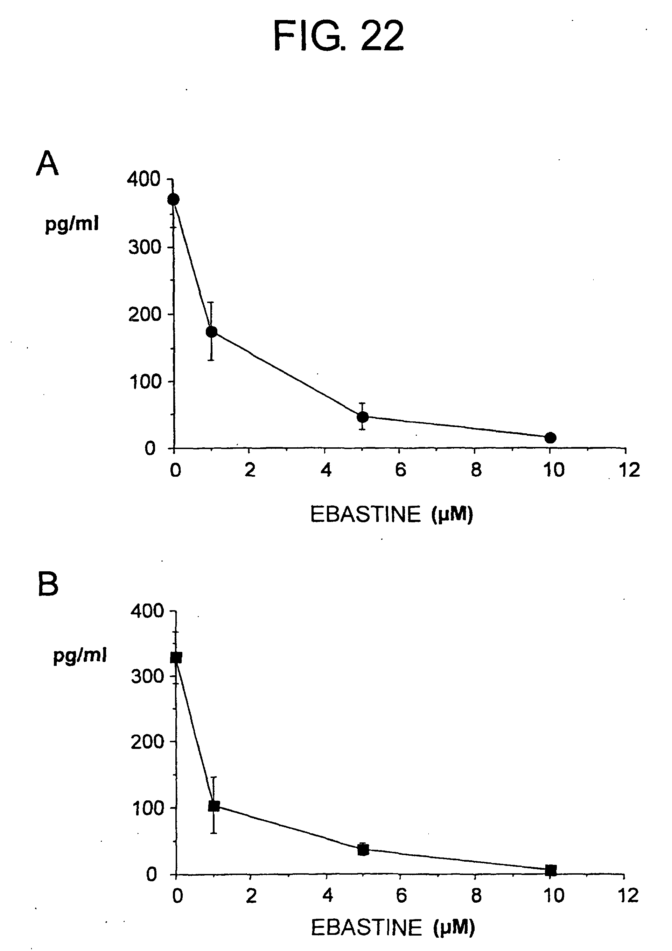

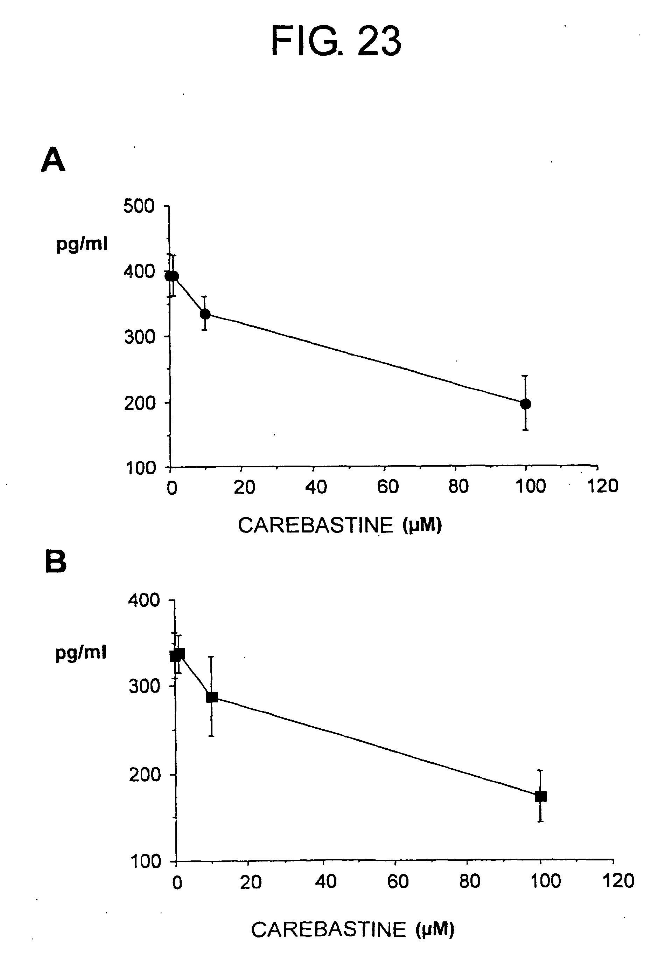

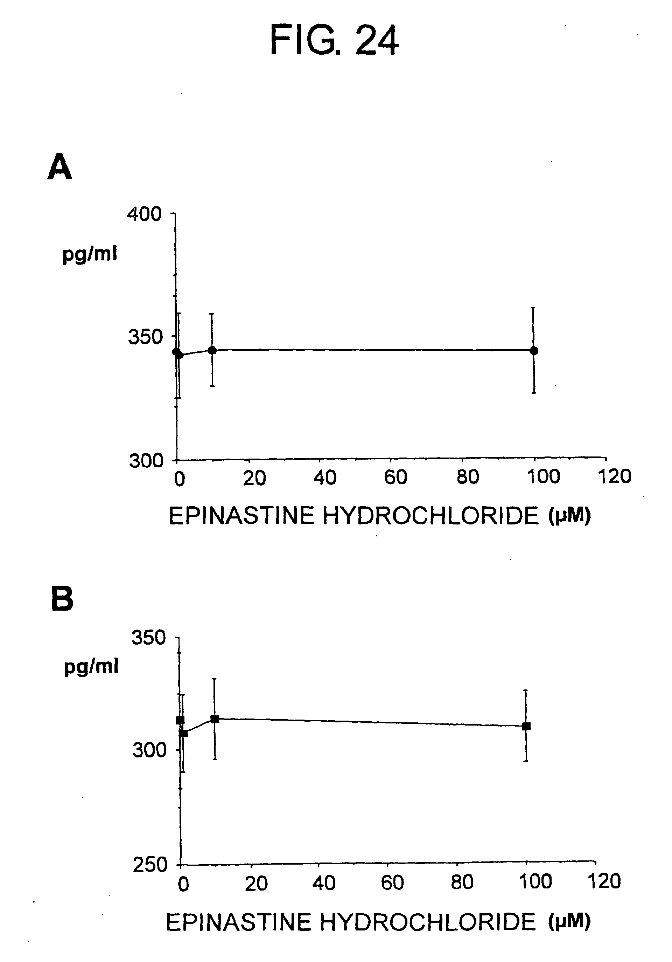

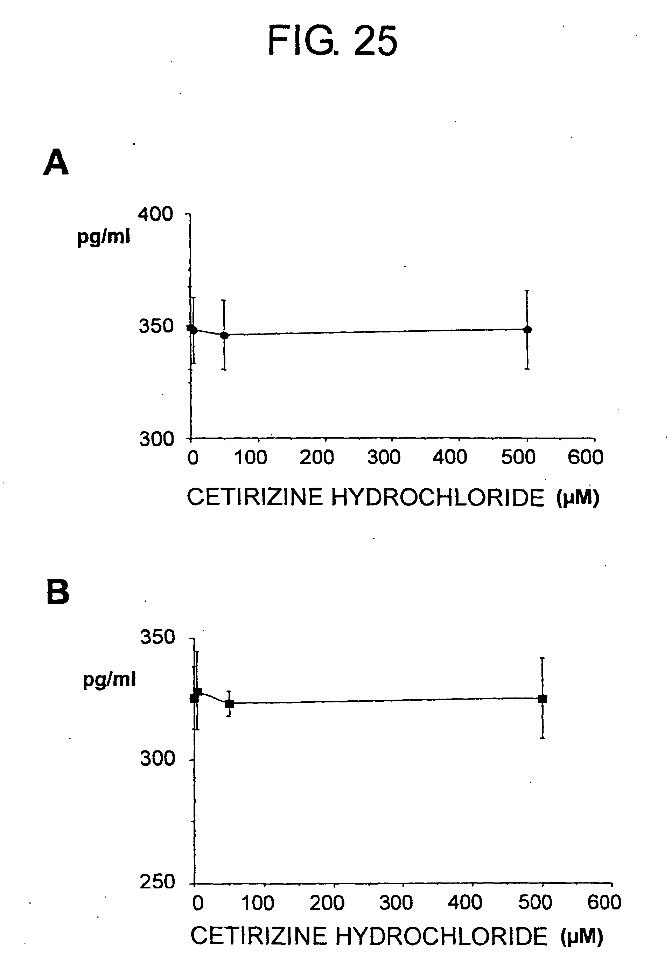

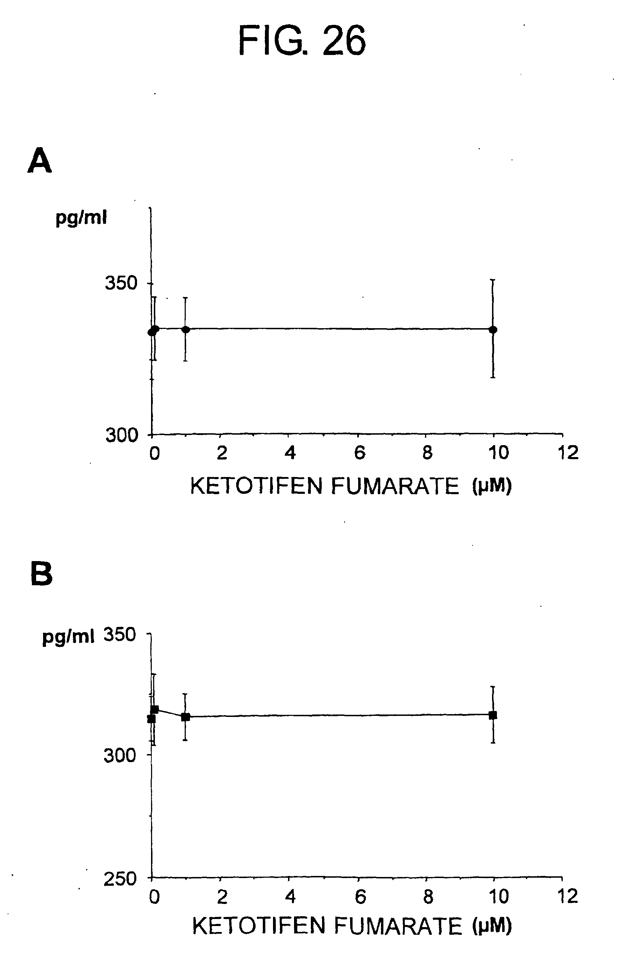

As shown in FIGs. 22 and 23, ebastine and carebastine suppressed, in a dose-dependent manner, the production of IL-6 and TNF-α from macrophages due to LPS stimulation. In particular, at an ebastine concentration of 1 µM, the production of the above cytokines was suppressed by 50% or more, and at 10 µM, the production was nearly completely inhibited (FIG. 22). Thus, ebastine was also demonstrated to inhibit the production of inflammatory cytokines such as IL-6 and TNF-α from macrophages. On the other hand, no inhibition on the production of IL-6 and TNF-α from macrophages due to LPS stimulation was observed with epinastine hydrochloride, cetirizine hydrochloride, and ketotifen fumarate (FIGs. 24 to 26).

### [Example 7] Suppression of Cas-L Tyrosine Phosphorylation in PHA-Stimulated T Cells by Ebastine

Ebastine suppressed T cell functions following T cell proliferation, such as cytokine production, cell migration, and adhesion, as well as the expression of β1 integrin, CD29. Thus, the present inventors examined the effects of ebastine on the tyrosine phosphorylation of CD29 integrin-related signal transduction molecules. PHA-stimulated T cells treated with ebastine (0.1 µM to 1.0 µM) were washed three times with Iscove's modified Dulbecco's medium (IMDM) and then solubilized by standing still for 30 minutes at 4°C in lysis buffer (1% NP40, 140 mM NaCl, 50 mM Tris, 5 mM EDTA, 5 mM NaF, 1 µg/ml aprotinin, 1 µg/ml leupeptin, 1 µg/ml pepstatin A, 1 mM PMSF, 1 mM sodium vanadate, pH 7.5). The cell lysate was centrifuged for 30 minutes at 15,000 rpm and 4°C to recover a cell extract from the supernatant. Tyrosine phosphorylation was analyzed by Western blotting of the cell extract using anti-phosphorylated tyrosine antibody. Among the bands obtained by the analysis of the cell extract, Cas-L, a signal transduction molecule downstream of β1 integrin, and FAK, involved in Cas-L phosphorylation, were analyzed by immunoprecipitation and Western blotting. Specifically, anti-Cas-L antibody or anti-FAK antibody was added to the above cell extract and reacted overnight at 4°C. Protein A beads were then added and allowed to react for four hours at 4°C. The reaction solution was centrifuged for five minutes at 3,000 rpm and 4°C, and the beads were washed with wash buffer (0.5% NP40, 140 mM NaCl, 50 mM Tris, 5 mM EDTA, 5 mM NaF, 1 µg/ml aprotinin, 1 µg/ml leupeptin, 1 µg/ml pepstatin A, 1 mM PMSF, 1 mM sodium vanadate, pH 7.5). This operation was repeated three times, sample buffer (1% SDS) was added to the beads from which the wash buffer was removed, and was reacted for five minutes at 95°C. The reaction solution was centrifuged and the supernatant was used as samples. An 8% acrylamide gel was prepared, and the samples were subjected to electrophoresis in an electrophoresis buffer (10% SDS, 250 mM Tris, 1.92 M glycine) for 120 minutes at 100V. After the electrophoresis, the bands were transferred using a transfer buffer (250 mM Tris, 1.92 M glycine, 20% methanol) on a PVDF membrane (Millipore) for 80 minutes at 100V and 4°C. After the transfer, the membrane was subjected to blocking in TBS-T (0.05% Tween20, 50 mM Tris, 150 mM NaCl, pH 7.5) supplemented with 5% milk for 60 minutes, and then primary antibody (1 µg/ml) was reacted in TBS-T for 60 minutes. After washing the membrane with TBS-T, HRP-labeled goat anti-mouse IgG antibody (1:10,000) was reacted as the secondary antibody for 60 minutes in TBS-T supplemented with 0.5% milk. The membrane was washed with TBS-T, reacted in ECL solution, and then exposed to a film for autoradiography.

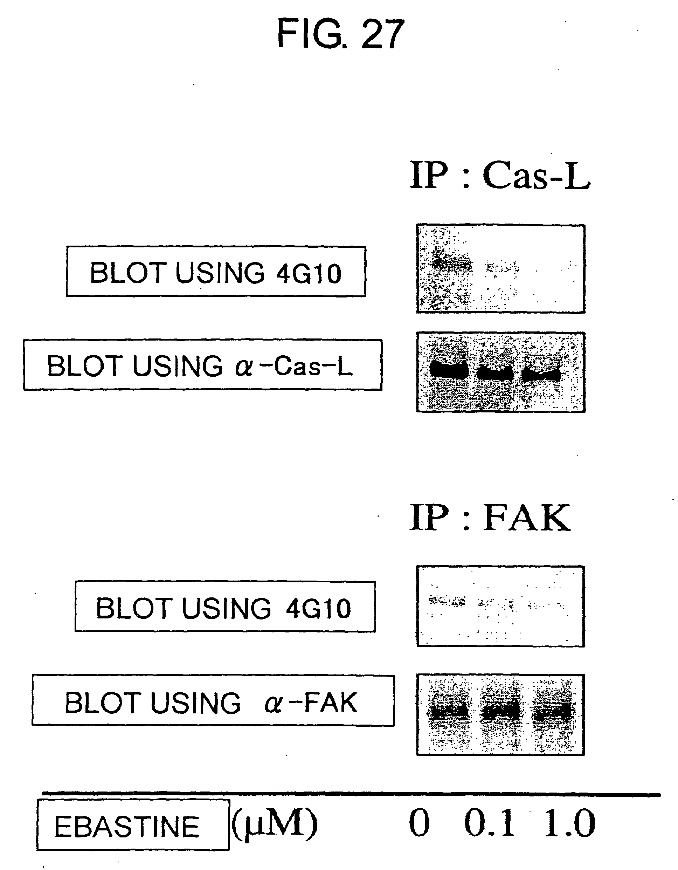

As a result, ebastine was found to slightly suppress the tyrosine phosphorylation of FAK in PHA-stimulated T cells, and to suppress the tyrosine phosphorylation of Cas-L in a dose dependent manner (FIG. 27). Thus, as one mechanism of the suppression of cell migration, ebastine was thought to suppress cell migration through the suppression of tyrosine phosphorylation of Cas-L.

As described above, ebastine inhibited the production of Th2 cytokines, IL-4 and IL-5, and the inflammatory cytokines IL-6 and TNF-α from T cells. This inhibition was specific, and did not influence the production of the Th1 cytokines, IL-2 and IFN-γ. In addition, ebastine also suppressed T cell migration, and further the production of inflammatory cytokines IL-6 and TNF-α from macrophages.

Ebastine is a novel H1 blocker that does not induce drowsiness, and utilizing this effect, has been clinically used for allergic rhinitis, urticaria, and the like (Ohtani H, Sato H, Iga T, Kotaki H, Sawada Y. "Pharmacokinetic-pharmacodynamic analysis of the arrhythmogenic potency of a novel antiallergic agent, ebastine, in rats." Biopharm Drug Dispose. 20:101-106, 1999; Matsuda M, Sakashita M, Mizuki Y, Yamaguchi T, Fujii T, Sekine Y. "Comparative pharmacokinetics of the histamine H1-receptor antagonist ebastine and its active metabolite carebastine in rats, guinea pigs, dogs and monkeys." Arzheim Forsch Drug Res. 44:55-59, 1994; Storms WW, 1966, *supra;* Kalis B, 1996, supra). Interactions between histamines and histamine H1 receptors induce pruritus and pain due to a series of physiological effects like contraction of important bronchial smooth muscles and increased vascular permeable stimulation of peripheral nerve endings, and also reflexively induce bronchial stenosis and coughing from stimulation of vagus nerve endings (Rimmer SJ, Church MK. "The pharmacology and mechanisms of action of histamine H1-antagonists." Clin Exp Allergy. 20 Suppl 2:3-17, 1990).

The effects of ebastine have been reported to be mainly due to the inhibition of the release of histamines from basophils and mast cells (Rimmer SJ, *et al*., 1990, supra; Birendia E. "Ebastine in context introduction." Drugs 52 Suppl 1:1-7, 1996). Moreover, ebastine has been reported to exhibit blocking of the release of anti-IgE-induced prostaglandin D2 and leukotriene C4/D4 from human nasal polyp cells after in vitro antigen administration (Campbell A, Michel F-B, Bremard-Qury C, Crampette L, Bousquet J. "Overview of allergic mechanisms. Ebastine has more than an antihistamine effect." Drugs 52 (Suppl 1):15-19, 1996). Furthermore, ebastine has been suggested to possess effects in addition to the blocking of histamine H1 receptors. Herein, effective novel effects of ebastatine are disclosed.

In hypersensitive responses, helper CD4 T cells play an important role in inducing allergic inflammatory responses. That is, cytokine production by Th2 type cells is essential to develop and maintain hypersensitive inflammatory responses (Kapsenberg M, *et al*., 1991, *supra;* Romagnani S., 1994, *supra*). The transportation of activated T cells to inflammatory sites is strictly controlled by a large number of molecules, particularly adhesive molecules and chemokines (Butcher EC and Picker LJ. "Lymphocyte homing and homeostasis." Science. 272:60-66, 1996). The selective recruiting of subsets of CD4⁺ effector T cells to inflammatory sites is thought to contribute to the development of different pathological symptoms. Activated Th2 type CD4 T cells that produce IL-4, IL-5, and IL-13 are detected in allergic inflammatory sites, bronchoalveolar lavage, and brachial biopsies of atopic and nonatopic asthmatics. Such cells have recently been positioned at the center of the action mechanism in the pathophysiology of asthma (Robinson DS *et al*. "Predominant Th2-type bronchoalveolar lavage T-lymphocyte population in atopic asthma." N Engl J Med. 326:298-304, 1992; Ying S *et al*. "Expression of IL-4 and IL-5 mRNA and protein products by CD4⁺ and CD8⁺ T cells, eosinophils and mast cells in bronchial biopsies obtained from atopic and nonatopic (intrinsic) asthmatics." J Immunol. 158:3539-3544, 1997). Therefore, Th2 type CD4 T cells and cytokines thereof are regarded as important targets for treatment. The Examples demonstrate that ebastine can inhibit, in a concentration-dependent manner, the production of Th2 type cytokines such as IL-4 and IL-5 due to various costimulation conditions.

On the other hand, ebastine does not affect the production of Th1 type cytokines such as IL-2 and IFN-γ. In addition, terfenadine which is a histamine H1 receptor antagonist having a chemical structure similar to that of ebastine has also been reported to have a similar effect (Munakata Y, Umezawa Y, Iwata S, Dong R-P, Yoshida S, Ishii T, Morimoto C. "Specific inhibition of Th2-type cytokine production from human peripheral T cells by terfenadine *in vitro."* Clin Exp Allergy. 29:1281-1286, 1999). However, since terfenadine induces occasionally QT interval elongation and infrequently fatal acute ventricular arrhythmia (Woolsey RL. "Cardiac actions of antihistamines." Ann Rev Pharmacol Toxicol. 36:233-252, 1996), it is currently not used in Europe, America, and Japan. On the other hand, ebastine does not have such severe cardiovascular side effects and has been reported not to affect the QT interval even if its dose is increased to five times from that used for the treatment (Ohtani H, et *al*., 1999, supra; Gillen MS, Miller B, Chaikin P, Morganroth J. "Effects of supratherapeutic doses of ebastine and terfenadine on the QT interval." Br J Clin Pharm. 52:201-204, 2001, Roberts DJ. "Assessing the cardiac safety of ebastine." Epilogue Drug Saf. 21 (Suppl I):89-92, 1999).

Terfenadine and other H1 receptor antagonists have been reported to inhibit eosinophil and neutrophil chemotaxis and reduce the expression of ICAM molecules on epithelial cell lines (Negro-Alvarez JM, Funes E, Garcia Canovas A, Herhamdez J, Garcia-Selles FJ, Pagan JA, Lopez-Sanchez JD. "Antiallergic properties of antihistamines." Allergol Immunopathol. 24:177-183, 1996). However, it has not been revealed whether these H1 antagonists inhibit T cell migration or not. On the other hand, as indicated by the above Examples, ebastine has been shown to inhibit T cell migration in a concentration-dependent manner. This suppression of T cell migration was due to the suppression of T cell adhesion to endothelial cells via suppressing the expression of adhesion-related T cell surface molecules CD26, CD29, and CD47. Furthermore, the research results of the present inventors also suggested suppression of tyrosine phosphorylation of Cas-L, which is a downstream signal of β1 integrin, as one of the mechanisms of the suppression of T cell migration. β1 integrin transmits a diversity of signals via the activation of various target molecules due to tyrosine phosphorylation, thereby exhibiting its biological functions. The tyrosine phosphorylation of the Cas-L positioned downstream of β1 integrin has been suggested to play an important role in the T cell activation and subsequent proliferation, cytokine production and cell adhesion, and the onset of cell migration (Ohashi Y. J Cell Biol, 1995, supra; Kamiguchi K, J Immunol, 1999, supra). Therefore, the suppression of Cas-L tyrosine phosphorylation by ebastine is strongly suggested as one of the suppression mechanisms of T cell migration.

Ebastine also inhibited the production of inflammatory cytokines such as IL-6 and TNF-α from human macrophages and T cells. Terfenadine has been reported to inhibit the release of IL-4 and IL-13 from human basophils (Gibbs BF, Vollrath IB, Albrecht C, Amon U, Wolff HH. "Inhibition of interleukin-4 and interleukin-13 release from immunologically activated human basophils due to the actions of anti-allergic drugs." Naunyn-Schmiedebergs Arch Pharmacol. 357:573-578, 1998), and ebastine has been reported to inhibit TNF-α, IL-8, and GM-CSF production from nasal polyp cells (Campbell A, et *al*. 1996, *supra*). However, the suppression of inflammatory cytokine production from macrophages and T cells as described above has not been reported.

Macrophages are dominant cells in human airways, and they are one of the most plentiful cells in the lung parenchyma of both healthy persons and asthmatics (Johnston RB Jr., 1988, *supra*), and provide the largest number of functional factors (Johnston RB Jr. "Current concepts: immunology. Monocytes and macrophages." N Engl J Med. 318:747, 1988). Macrophages synthesize and release a wide range of inflammatory factors such as oxygen, cytokines, and chemokines (Nathan CF "Secretary products of macrophages." J Clin Invest 79:319, 1987). The main chemokines produced from lung macrophages are IL-6 and TNF-α (Marone G, Gentile M, Petraroli A, De Rosa N, Triggiani M. "Histamine-induced activation of human lung macrophages." Int Arch Allergy Immunol. 124:249, 2001; Amrani Y, Chen H, Panettieri RA Jr. "Activation of tumor necrosis factor receptor 1 in airway smooth muscle: a potential pathway that modulates bronchial hyper-responsiveness in asthma." Respir Res. 1:49, 2000).

According to a recent study, since tumor necrosis factor (TNF)-α is produced in large amounts in the airways of asthmatics, it is suggested to be involved in the onset of bronchial hypersensitive responses by directly changing the state of airway smooth muscle (ASM) (Amrani Y, Chen H, Panettieri RA Jr. "Activation of tumor necrosis factor receptor 1 in airway smooth muscle: a potential pathway that modulates bronchial hyper-responsiveness in asthma." Respir Res. 1:49, 2000).

In addition, IL-6 is a cytokine with many functions that is released in inflammatory diseases. This cytokine is involved in the differentiation and maturation of human mast cells, and in the control of IgE synthesis (Hirano T. "Interleukin 6 and its receptor: ten years later." Int Rev Immunol. 16:249, 1998). Elevated IL-6 levels have been detected in blood, bronchoalveolar lavage fluid after bronchial treatment of asthmatics, and in bronchial biopsies, which suggests elevated IL-6 expression (Virchow JC Jr., Walker C, Hafner D, Kortsik C, Werner P, Matthys H, *et al*. "T cells and cytokines in bronchoalveolar lavage fluid after segmental allergen provocation in atopic asthma." Am J Respir Crit Care Med. 151:960, 1995).

Cytokine networks and abnormalities thereof in autoimmune diseases are attracting attention in recent years. One factor of the onset of autoimmune diseases is thought to be the Th1/Th2 imbalance induced by the invasion of autoantigens or foreign antigens. Autoimmune diseases can be broadly classified into Th1-dependent types, Th2-dependent types, and those in which both are involved (Ohta A, Nishimura T. "Cytokine networks and abnormalities thereof in autoimmune diseases." The Medical Frontline (in Japanese) 53 (6) : 1338, 1998). As representative diseases, SLE is Th2 cytokine-dominant while chronic rheumatoid arthritis involves both Th1 and Th2 cytokines (Ohta A, The Medical Frontline, 1998, *supra*).

Based on the various findings and the Examples given above, suppression of IL-6 and TNF-α production from T cells and macrophages by ebastine is suggested to be an important target for asthma treatment.

Furthermore, the antiallergic action of epinastine hydrochloride is thought to involve the suppression of the production of Th1 and Th2 type cytokines from T cells in addition to histamine antagonism.

In addition to its antihistamine effects, ebastine has been shown to have novel functional effects on T cells and macrophages. Ebastine inhibited Th2 type cytokine production from T cells, T cell migration, and production of inflammatory cytokines (IL-6, TNF-α, etc.) from T cells and macrophages. Therefore, ebastine may serve as an essential agent for the treatment of immunoallergic diseases caused by T cells, including asthma, atopic dermatitis, Th2 type autoimmune diseases, systemic lupus erythematosus, myasthenia gravis, chronic GVHD, inflammatory diseases such as Crohn's disease, and so on. Moreover, chronic rheumatoid arthritis is triggered by the production of inflammatory cytokines such as TNF-α and IL-6 from T cells and macrophages at inflammatory sites, and T cell migration to inflammatory sites. Therefore, ebastine may be applicable for the treatment of these diseases. In particular, recent reports showed that anti-TNF-α treatments are effective on rheumatic arthritis and Crohn's disease (Taylor PC, Williams RO, Maini RN. "Immunotherapy for rheumatoid arthritis." Curr Opin Immunol. 13:611, 2001; Kam LY, Targan SR. "TNF-alpha antagonists for the treatment of Crohn's disease." Expert Opin Pharmacother. 1:615, 2000) and that IL-6 receptor antibody treatments are also effective in the treatment of rheumatoid arthritis (Choy EH, Isenberg DA, Garrood T, Farrow S, Ioannou Y, Bird H, *et al*. "Therapeutic benefit of blocking interleukin-6 activity with an anti-interleukin-6 receptor monoclonal antibody in rheumatoid arthritis: A randomized, double-blind, placebo-controlled, dose-escalation trial." Arthritis Rheum. 46(12):3143, 2002). Thus, ebastine may be used in the treatment of rheumatoid arthritis.

On the other hand, epinastine hydrochloride is reported to have an efficacy of 47.0% in the improvement of bronchial asthma to a medium degree or greater, and its administration for psoriasis vulgaris accompanying pruritus has been clinically approved (according to the Interview Form for epinastine hydrochloride). Regarding the clinical effects of epinastine hydrochloride, in addition to the antagonistic effect to H1 receptors, it is reported to influence the cytokine production of peripheral blood eosinophils and mononuclear cells (Sagara H. "Effects of epinastine on eosinophils." Allergology (in Japanese). 12(6):587, 2001; Kojima Y, Yoshikawa Y, Nishibe A, Kaneko F. "Effects of antiallergic agents on cytokine production by peripheral blood mononuclear cells in atopic dermatitis patients." Jpn J Dermatology. 106(4):395, 1996). According to the present invention, epinastine hydrochloride was shown to suppress T cell proliferation and Th1 and Th2 cytokine production from T cells. This is thought to be one mechanism explaining its efficacy, since psoriasis vulgaris in particular is assumed to be a Th1 type disease and epinastine hydrochloride is currently used against it.

However, H1 blockers cetirizine hydrochloride and ketotifen fumarate were not found to affect the functions of T cells and macrophages.

According to a comparison of the IC₅₀ values of inhibiting the ligand binding of H1 blockers to H1 receptors using Chinese Hamster Ovary cells that express human recombinant H1 receptors, the value of ketotifen fumarate was 0.52±0.06 nM, that of epinastine hydrochloride was 1.1±0.16 nM, and that of cetirizine hydrochloride was 11±0.67 nM (Nonaka H *et al*., 1998, *supra*). In addition, in a comparison of the IC₅₀ values of the effects on histamine-induced contraction in trachea samples excised from guinea pigs, the value of ketotifen fumarate was 0.0025 µM, that of carebastine was 0.12 µM, and that of ebastine was >10 µM (according to the Interview Form for ebastine). As described above, among the H1 blockers used herein, ketotifen fumarate had the strongest affinity and antagonism to an H1 receptor. However, ketotifen fumarate showed no effect on T cells and macrophages. Thus, H1 blockers were suggested to exert effects on immune cells through other than antihistaminic effects on histamine receptors.

In addition to H1 receptors, H1 blockers also bind to H2 receptors although to a lesser extent. The IC₅₀ values (nM) in the inhibition of ligand binding to H2 receptors was 640±62, 2200±450, and >10,000 for epinastine hydrochloride, ketotifen fumarate, and cetirizine hydrochloride respectively (Nonaka H *et al*., 1998, *supra*). The above report was based on non-clinical experiments performed to supplement the fact that the binding specificity of cetirizine hydrochloride to H1 receptors as an H1 blocker is higher than that of epinastine hydrochloride and ketotifen fumarate. In this report, no control experiments to ebastine were performed. Binding to H2 receptors is currently considered as only a side effect of H1 blockers, and thus, details regarding the binding of ebastine to H2 receptors have not been reported. However, the comparison of the IC₅₀ values of the effects on histamine-induced contraction in trachea samples excised from guinea pigs and such showed that the affinity of ebastine (or carebastine) to H1 receptors is much less than that of ketotifen fumarate. Therefore, the specificity of ebastine (or carebastine) to H1 receptors may be smaller than ketotifen fumarate.

Histamine receptors in human T cells are mainly H2 receptors. The present inventors previously reported that the H1 blocker terfenadine suppresses cytokine production by T cells (Munakata et *al*., 1999). In the binding experiment to H2 receptors using terfenadine, the inhibition rate to [³H] -tiotidine was 29% at maximum concentration (Uchida M, Omi N, Koga Y, Morooka S. "Pharmacological effects of cetirizine." The Clinical Report (in Japanese). 28(7):1795-1812, 1994). According to the present invention, suppression of T cell cytokine production was observed for the H1 blockers ebastine and epinastine hydrochloride, which have a relatively low specificity to H1 receptors. This may be due to the fact that they acted on H2 receptors on human T cells. In mice, histamine has been reported to enhance Th1 cytokine production via H1 receptors, and conversely suppress both Th1 and Th2 cytokine production via H2 receptors (Jutel M, *et al*., Nature, 2001, *supra*). According to the present invention, the effect of suppressing cytokine production via H2 receptor stimulation demonstrated in mouse T cell systems was suggested to also occur in human T cells.

### Industrial Applicability

As described above, ebastine and carebastine have been shown to inhibit T cell proliferation, Th2 type cytokine production, inflammatory cytokine production, and T cell migration under costimulation conditions, all in a concentration-dependent manner. Due to these effects, ebastine and carebastine are expected to serve as essential agents for the treatment of immunoallergic diseases caused by T cells. Examples of diseases include asthma, atopic dermatitis, Th2 type autoimmune diseases, and inflammatory diseases such as systemic lupus erythematosus, myasthenia gravis, chronic GVHD, Crohn's disease, and chronic rheumatoid arthritis.

In addition, epinastine hydrochloride has been shown to inhibit T cell proliferation, Th2 type cytokine production, and Th1 type cytokine production, all in a concentration-dependent manner. Due to these effects, epinastine hydrochloride is expected to serve as an essential agent for the treatment of diseases such as allergy, asthma, psoriasis vulgaris, organ-specific autoimmune diseases involving Th1/Th2 cytokine imbalances (autoimmune thyropathy, and multiple sclerosis), inflammatory diseases (Crohn's disease, inflammatory bowel disease, and rheumatoid arthritis), systemic autoimmune diseases (systemic lupus erythematosus, and myasthenia gravis), and chronic GVHD.

## Claims

1. A compound having the formula (1): 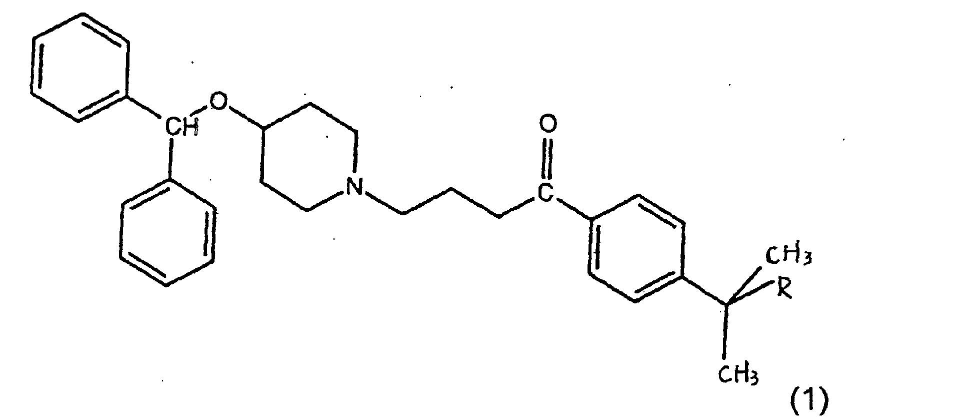 (wherein, R is CH₃ or COOH),
that is able to suppress an allergic immune response involving an immunocyte, wherein said allergic immune response is selected from the group consisting of:
(A) proliferation of T cell;
(B) production of Th2 type cytokine;
(C) production of inflammatory cytokine; and
(D) T cell migration.

2. A compound having the formula (2) or salt thereof: 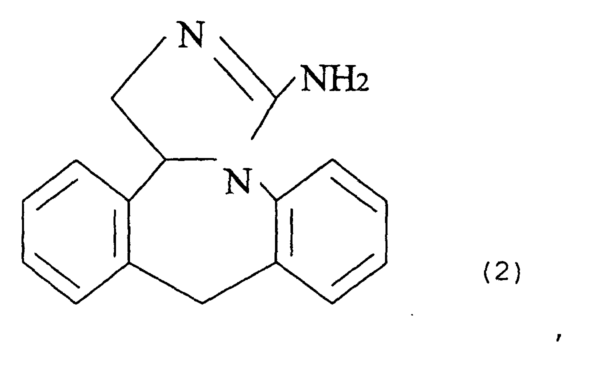 that is able to suppress an allergic immune response involving an immunocyte, wherein said allergic immune response is selected from the group consisting of:
(A) proliferation of T cell;
(B) production of Th2 type cytokine; and
(C) production of Th1 cytokine.

3. The compound according to claim 1, wherein the inflammatory cytokine is produced from a T cell or macrophage.

4. A composition comprising the compound according to claim 1 or 3.

5. A composition comprising the compound or salts thereof according to claim 2.

6. An agent for suppressing an allergic immune response involving an immunocyte that comprises, as an active ingredient, the compound having the formula (1): 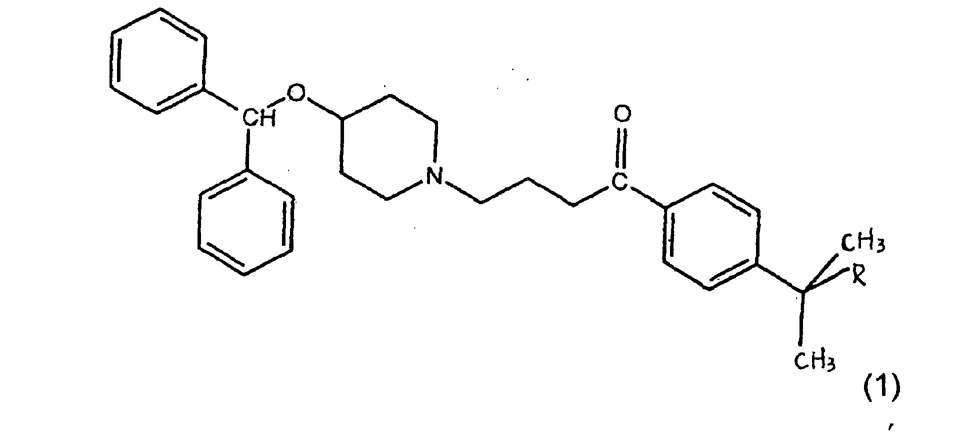 wherein said allergic immune response is selected from the group of:
(A) proliferation of T cell;
(B) production of Th2 type cytokine;
(C) production of inflammatory cytokine; and
(D) T cell migration.

7. An agent for suppressing an allergic immune response involving an immunocyte that comprises, as an active ingredient, the compound having the formula (2) or salts thereof: 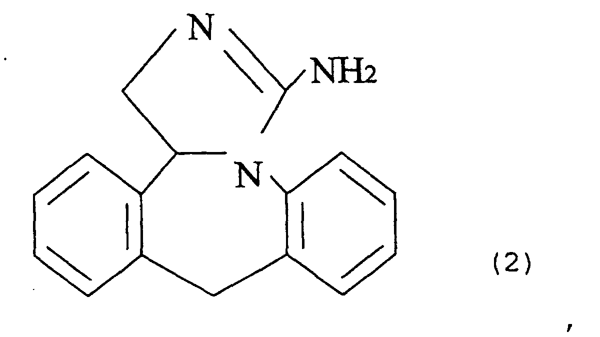 wherein said allergic immune response is selected from the group of:
(A) proliferation of T cell;
(B) production of Th2 type cytokine; and
(C) production of Th1 type cytokine.
